# EUROPEAN PATENT APPLICATION

(11) **EP 4 748 935 A1**
(43) Date of publication of application: **27.05.2026**
(21) Application number: 24848949.4
(22) Date of filing: 19.07.2024
(51) Int. Cl.: C12N 15/31, C12N 1/20, C12N 1/21, C12N 15/63

(54) **COMPOSITION FOR IMPARTING RESISTANCE TO ENVIRONMENTAL STRESS TO BACTERIUM**

(30) Priority: 28.07.2023 JP 2023123842
(71) Applicant: Kabushiki Kaisha Yakult Honsha, Tokyo, 105-8660 (JP)
(72) Inventor: KATO Kosuke, Tokyo 105-8660 (JP); NAKAMURA Madoka, Tokyo 105-8660 (JP); SERATA Masaki, Tokyo 105-8660 (JP); OKUMURA Takekazu, Tokyo 105-8660 (JP)
(74) Representative: Mewburn Ellis LLP
(86) International application number: PCT/JP2024/025886
(87) International publication number: WO 2025/028294

(57) **Abstract**

A technique is provided for imparting environmental stress tolerance to bacteria such as lactic acid bacteria. There is provided a composition for imparting environmental stress tolerance to bacteria, the composition containing a cps1 gene cluster derived from *Lacticaseibacillus paracasei.* This composition is ideally used in order to impart tolerance to environmental stress caused by at least one selected from the group consisting of acids, bile components, alcohols, metal ions, hydrophobic substances, and surfactants.

## Description

### [TECHNICAL FIELD]

The present invention relates to the use of a gene cluster for forming a cell-wall polysaccharide in lactic acid bacteria.

### [BACKGROUND ART]

In the prior art, probiotics that promote good health through ingestion of live lactic acid bacteria have been proposed. It has been reported that certain probiotics alleviate symptoms of upper respiratory tract infections due to the action of the ingested lactic acid bacteria (Non-patent Documents 1 and 2).

However, in recent years, there have been attempts to utilize microbial-cell-body-derived components as functional components for a subject. For example, Patent Documents 1 and 2 indicate that cell-wall-derived polysaccharides present in the cell walls of lactic acid bacteria have functionality for regulating the production of cytokines, such as IL6 and IL12, that are produced by immunoregulatory cells. Non-patent Documents 3 to 6 have made it clear that high-molecular-weight polysaccharides having a molecular weight exceeding 100,000 are the molecular entity of the cells of lactic acid bacteria or the cell-wall polysaccharides derived therefrom having such functionalities.

### [Related Art Documents]

### [Patent Documents]

[Patent Document 1] Japanese Laid-open Patent Application No. 2003-73286
[Patent Document 2] Japanese Laid-open Patent Application No. 2015-129114

### [Non-patent Documents]

[Non-patent Document 1] Kan Shida, Tadashi Sato, Ryoko Iizuka, Ryotaro Hoshi, Osamu Watanabe, Tomoki Igarashi, Kouji Miyazaki, Masanobu Nanno, and Fumiyasu Ishikawa. "Daily intake of fermented milk with Lactobacillus casei strain Shirota reduces the incidence and duration of upper respiratory tract infections in healthy middle-aged office workers." Eur. J. Nutr. (2017), 56:45-53.

[Non-patent Document 2] Michael Gleeson, Nicolette C. Bishop, Marta Oliveira, and Pedro Tauler. "Daily Probiotic's (Lactobacillus casei Shirota) Reduction of Infection Incidence in Athletes." International Journal of Sport Nutrition and Exercise Metabolism, 2010, 1-10.

[Non-patent Document 3] Masato Nagaoka, Masami Muto, Koji Nomoto, Takesi Matuzaki, Tunekazu Watanabe, and Teruo Yokokura. "Structure of Polysaccharide-Peptidoglycan Complex from the Cell Wall of Lactobacillus casei YIT9018." J. Biochemistry (1990), 108(4), pp. 568-571.

[Non-patent Document 4] S. Matsumoto, T. Hara, T. Hori, K. Mitsuyama, M. Nagaoka, N. Tomiyasu, A. Suzuki, and M. Sata. "Probiotic Lactobacillus-induced improvement in murine chronic inflammatory bowel disease is associated with the down-regulation of pro-inflammatory cytokines in lamina propria mononuclear cells." Clin. Exp. Immunol. (2005), 140(3), pp. 417-426.

[Non-patent Document 5] S. Matsumoto, T. Hara, M. Nagaoka, A. Mike, K. Mitsuyama, T. Sako, M. Yamamoto, S. Kado, and T. Takada. "A component of polysaccharide peptidoglycan complex on Lactobacillus induced an improvement of murine model of inflammatory bowel disease and colitis-associated cancer." Immunology (2008), 128, e170-e180.

[Non-patent Document 6] Emi Yasuda, Masaki Serata, and Tomoyuki Sako. "Suppressive Effect on Activation of Macrophages by Lactobacillus casei Strain Shirota Genes Determining the Synthesis of Cell-Wall-Associated Polysaccharides." Appl. Environ. Microbiol. (2008), 74(15), pp. 4746-4755.

### [DISCLOSURE OF THE INVENTION]

### [Problems the Invention is Intended to Solve]

In the field of probiotics, a problem is presented in that the activity of lactic acid bacteria or the integrity of the cells thereof could be compromised by environmental stress such as exposure of the bacteria to low pH during growth or at the time of ingestion.

It is an object of the present invention to provide technology for imparting environmental stress tolerance to bacteria such as lactic acid bacteria.

### [Means for Solving the Aforementioned Problems]

As a result of thorough investigations for the purpose of achieving the aforementioned object, the inventors perfected the present invention upon discovering that a gene cluster associated with formation of cell-wall polysaccharides in lactic acid bacteria has an action for imparting environmental stress tolerance to bacteria.

Specifically, according to a first aspect, the present invention provides a composition for imparting environmental stress tolerance to bacteria, the composition including a cps1 gene cluster derived from *Lacticaseibacillus paracasei.*

In the composition according to the first aspect of the present invention, it may be preferable that the cps1 gene cluster includes a cps1A gene, a cps1B gene, a cps1C gene, a cps1D gene, a cps1E gene, a cps1F gene, a cps1G gene, a cps1H gene, a cps1I gene, and a cps1J gene.

In the composition according to the first aspect of the present invention, it may be optionally that:
the cps1A gene has the polynucleotide sequence indicated by SEQ ID NO: 1 or a polynucleotide sequence that is at least 90% homologous thereto,
the cps1B gene has the polynucleotide sequence indicated by SEQ ID NO: 2 or a polynucleotide sequence that is at least 90% homologous thereto,
the cps1C gene has the polynucleotide sequence indicated by SEQ ID NO: 3 or a polynucleotide sequence that is at least 90% homologous thereto,
the cps1D gene has the polynucleotide sequence indicated by SEQ ID NO: 4 or a polynucleotide sequence that is at least 90% homologous thereto,
the cps1E gene has the polynucleotide sequence indicated by SEQ ID NO: 5 or a polynucleotide sequence that is at least 90% homologous thereto,
the cps1F gene has the polynucleotide sequence indicated by SEQ ID NO: 6 or a polynucleotide sequence that is at least 90% homologous thereto,
the cps1G gene has the polynucleotide sequence indicated by SEQ ID NO: 7 or a polynucleotide sequence that is at least 90% homologous thereto,
the cps1H gene has the polynucleotide sequence indicated by SEQ ID NO: 8 or a polynucleotide sequence that is at least 90% homologous thereto,
the cps1I gene has the polynucleotide sequence indicated by SEQ ID NO: 9 or a polynucleotide sequence that is at least 90% homologous thereto, and
the cps1J gene has the polynucleotide sequence indicated by SEQ ID NO: 10 or a polynucleotide sequence that is at least 90% homologous thereto.

In the composition according to the first aspect of the present invention, it may be optionally that:
the cps1A gene codes the amino acid sequence indicated by SEQ ID NO: 11 or an amino acid sequence that is at least 90% homologous thereto,
the cps1B gene codes the amino acid sequence indicated by SEQ ID NO: 12 or an amino acid sequence that is at least 90% homologous thereto,
the cps1C gene codes the amino acid sequence indicated by SEQ ID NO: 13 or an amino acid sequence that is at least 90% homologous thereto,
the cps1D gene codes the amino acid sequence indicated by SEQ ID NO: 14 or an amino acid sequence that is at least 90% homologous thereto,
the cps1E gene codes the amino acid sequence indicated by SEQ ID NO: 15 or an amino acid sequence that is at least 90% homologous thereto,
the cps1F gene codes the amino acid sequence indicated by SEQ ID NO: 16 or an amino acid sequence that is at least 90% homologous thereto,
the cps1G gene codes the amino acid sequence indicated by SEQ ID NO: 17 or an amino acid sequence that is at least 90% homologous thereto,
the cps1H gene codes the amino acid sequence indicated by SEQ ID NO: 18 or an amino acid sequence that is at least 90% homologous thereto,
the cps1I gene codes the amino acid sequence indicated by SEQ ID NO: 19 or an amino acid sequence that is at least 90% homologous thereto, and
the cps1J gene codes the amino acid sequence indicated by SEQ ID NO: 20 or an amino acid sequence that is at least 90% homologous thereto.

In the composition according to the first aspect of the present invention, it may be optionally that the cps1 gene cluster is used in a form of being incorporated into a vector.

In the composition according to the first aspect of the present invention, it may be optionally that the environmental stress against which tolerance is to be imparted is caused by at least one selected from the group consisting of acids, bile components, alcohols, metal ions, hydrophobic substances, and surface-active agents.

According to a second aspect, the present invention provides a method for imparting environmental stress tolerance to bacteria, the method being characterized by including a step in which, by using the aforementioned composition, the cps1 gene cluster is introduced into designated bacteria.

In the method according to the second aspect of the present invention, the method includes imparting tolerance to environmental stress caused by at least one selected from the group consisting of acids, bile components, alcohols, metal ions, hydrophobic substances, and surface-active agents.

### [Effect of the Invention]

According to the present invention, it is possible to provide a composition and a method that make it possible to impart environmental stress tolerance to bacteria by using a gene cluster associated with formation of cell-wall polysaccharides in lactic acid bacteria.

### [BRIEF DESCRIPTION OF THE DRAWINGS]

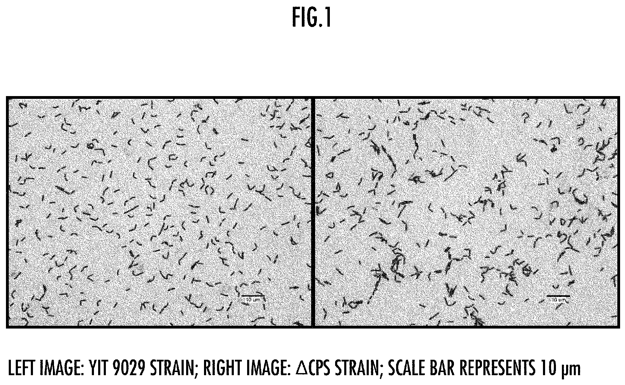

FIG. 1 is a set of illustrations showing examples of observation images obtained when cells of the YIT 9029 strain and the Δcps strain are subjected to gram staining and observed under a microscope in test example 1;

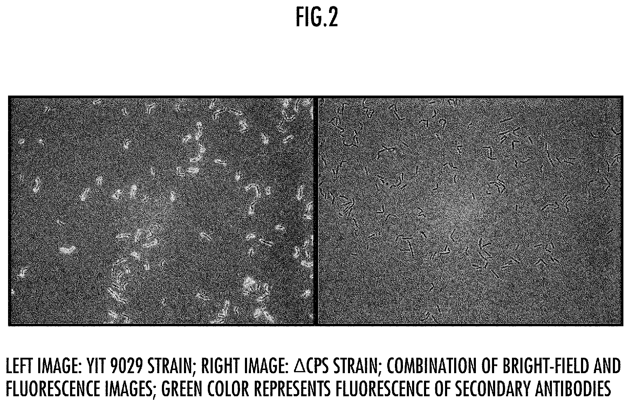

FIG. 2 is a set of illustrations showing examples of fluorescence microscope observation images obtained from investigating the antibody reactivity of the cells of the YIT 9029 strain and the Δcps strain in test example 2;

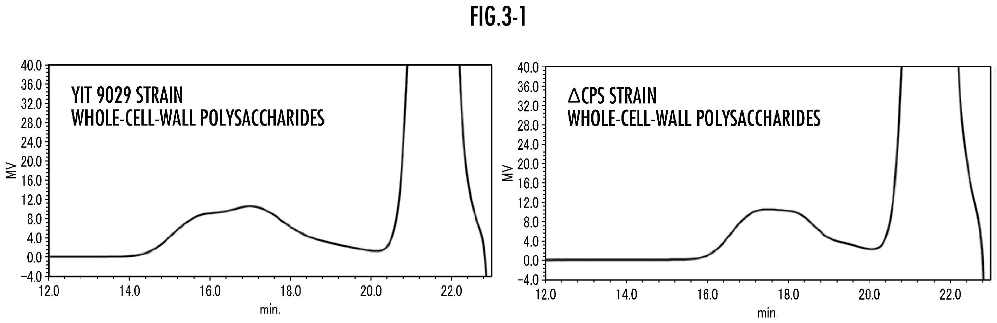

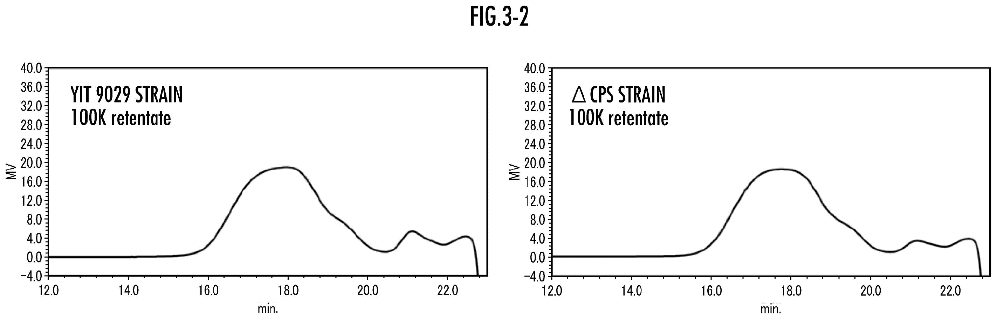

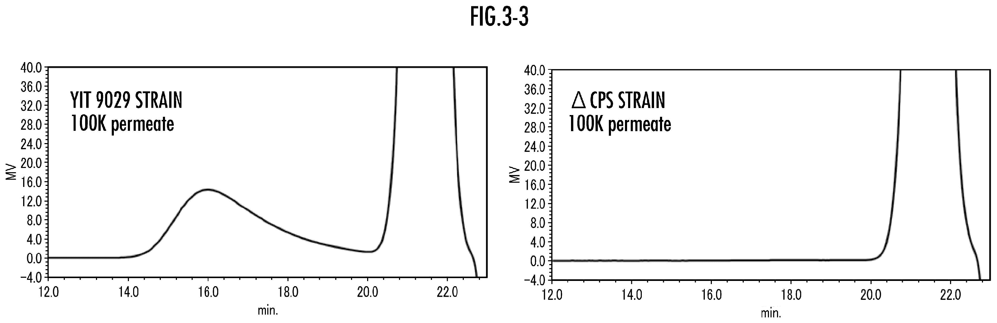

FIG. 3 is a set of graphs showing results from when a whole-cell-wall polysaccharide solution prior to ultrafiltration, a retentate after ultrafiltration, and a permeate after ultrafiltration are each separated and analyzed through gel filtration column chromatography in test example 3;

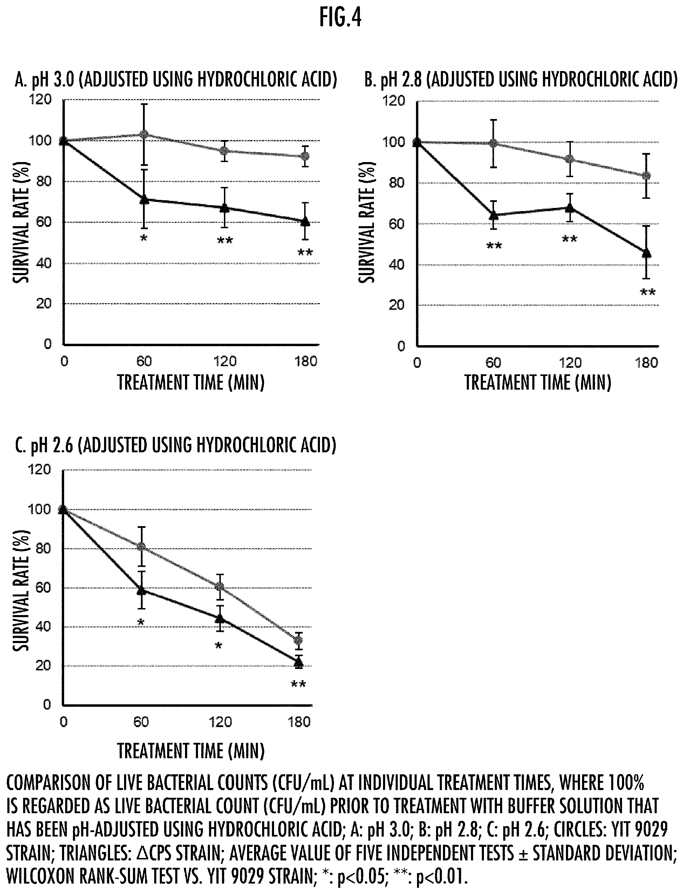

FIG. 4 is a set of graphs showing results from investigating the acid tolerance of the YIT 9029 strain and the Δcps strain in test example 4, said graphs showing the survival rate over time after each treatment, when these strains were treated with a low-pH buffer solution of which the pH was adjusted using hydrochloric acid;

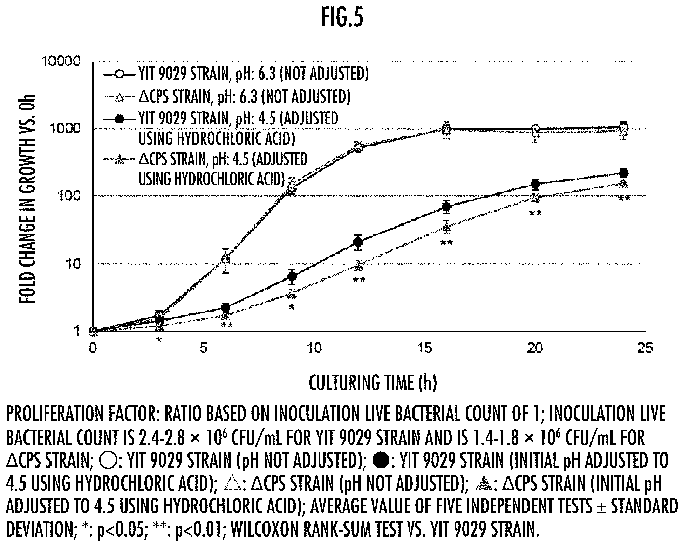

FIG. 5 is a graph showing results from investigating the acid tolerance of the YIT 9029 strain and the Δcps strain in test example 5, said graph showing a transition in the fold change in bacterial growth over time when these strains were cultured using a non-pH-adjusted culture medium or a low-pH culture medium (pH: 4.5) of which pH was adjusted by adding hydrochloric acid;

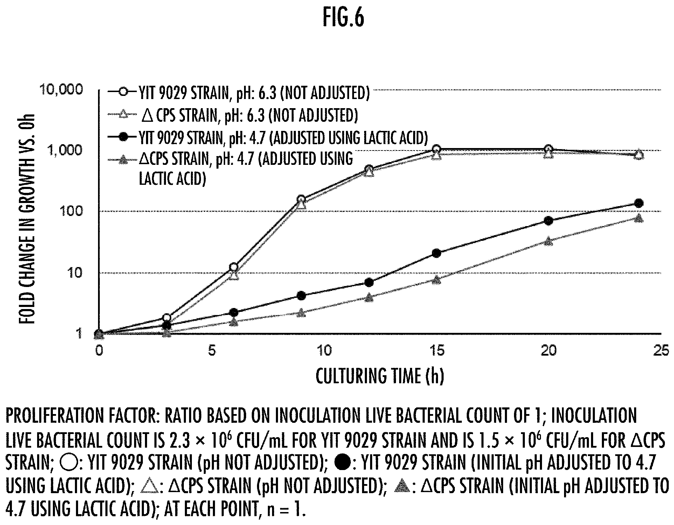

FIG. 6 is a graph showing results from investigating the acid tolerance of the YIT 9029 strain and the Δcps strain in test example 5, said graph showing a transition in the fold change in bacterial growth over time when these strains were cultured using a non-pH-adjusted culture medium or a low-pH culture medium (pH: 4.7) of which pH was adjusted by adding lactic acid;

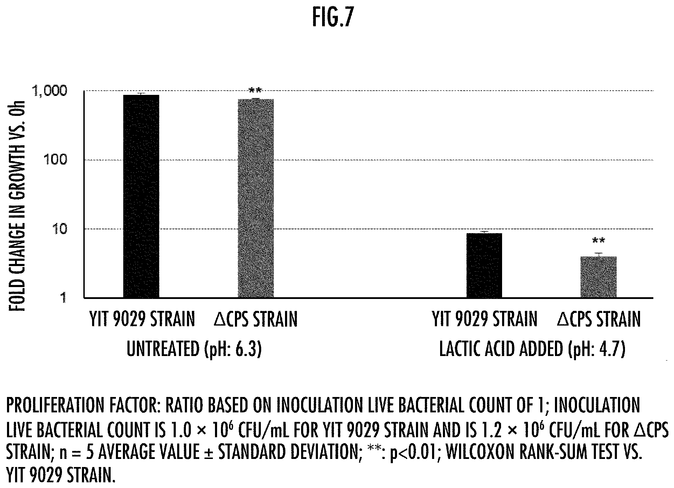

FIG. 7 is a bar graph showing results from investigating the acid tolerance of the YIT 9029 strain and the Δcps strain in test example 5, said bar graph showing results pertaining to the fold change in bacterial growth after 12 hours of culture using a non-pH-adjusted culture medium or a low-pH culture medium (pH: 4.7) of which pH was adjusted by adding lactic acid;

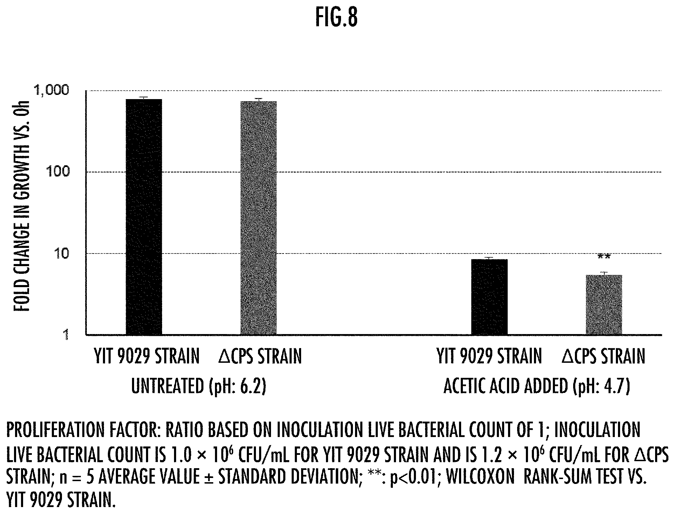

FIG. 8 is a bar graph showing results from investigating the acid tolerance of the YIT 9029 strain and the Δcps strain in test example 5, said bar graph showing results pertaining to the fold change in bacterial growth after 12 hours of culture using a non-pH-adjusted culture medium or a low-pH culture medium (pH: 4.7) of which pH was adjusted by adding acetic acid;

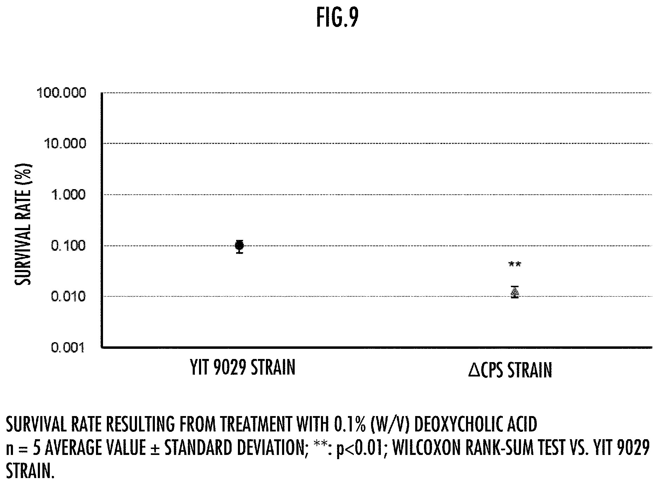

FIG. 9 is a graph showing results from investigating the bile tolerance of the YIT 9029 strain and the Δcps strain in test example 6, said graph showing results pertaining to the survival rate when these strains were treated for 5 minutes with PBS (pH: 7.0) containing deoxycholic acid at a concentration of 0.1% (w/v);

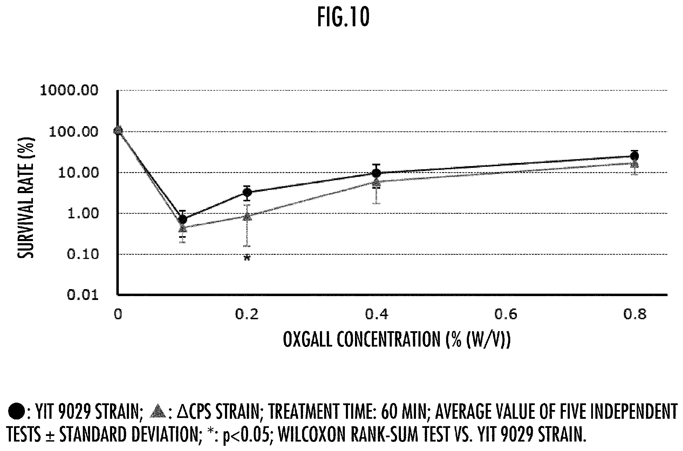

FIG. 10 is a graph showing results from investigating the bile tolerance of the YIT 9029 strain and the Δcps strain in test example 6, said graph showing results pertaining to the survival rate when these strains were treated for 60 minutes with PBS (pH: 7.0) containing bovine bile powder at the indicated concentrations;

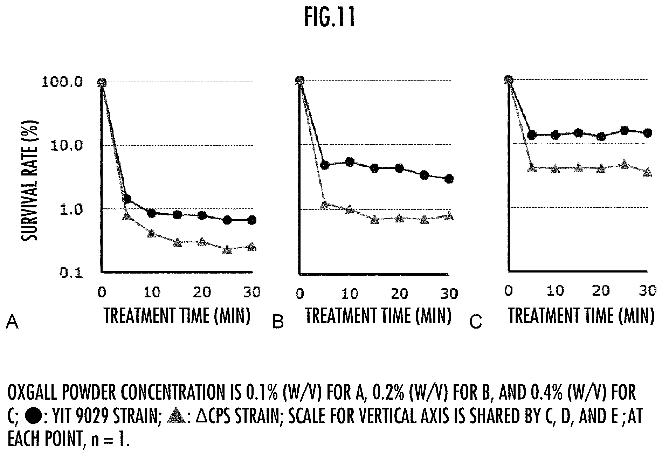

FIG. 11 is a set of graphs showing results from investigating the bile tolerance of the YIT 9029 strain and the Δcps strain in test example 6, said graphs showing results pertaining to the survival rate over time after these strains were treated with PBS (pH: 7.0) containing bovine bile powder at the indicated concentrations;

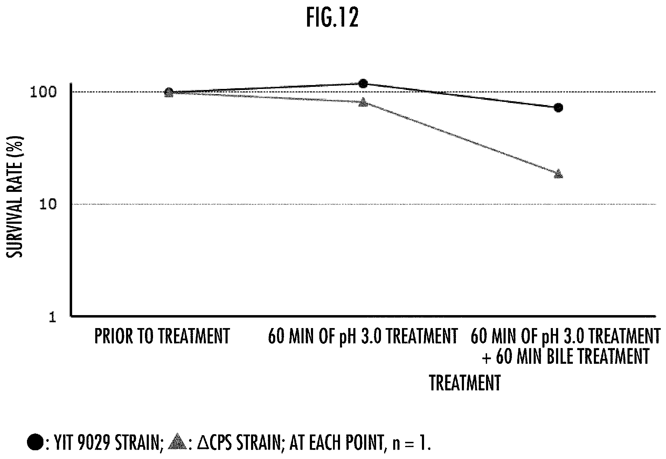

FIG. 12 is a graph showing results from investigating the acid tolerance and the bile tolerance of the YIT 9029 strain and the Δcps strain in test example 7, said graph showing results pertaining to the survival rate when these strains were treated for 60 minutes with a low-pH buffer solution (pH: 3.0) that was prepared using hydrochloric acid, or pertaining to the survival rate when these strains were consecutively treated with PBS (pH: 7.0) containing bovine bile powder at a concentration of 0.2% (w/v) after treatment with the said low-pH buffer solution;

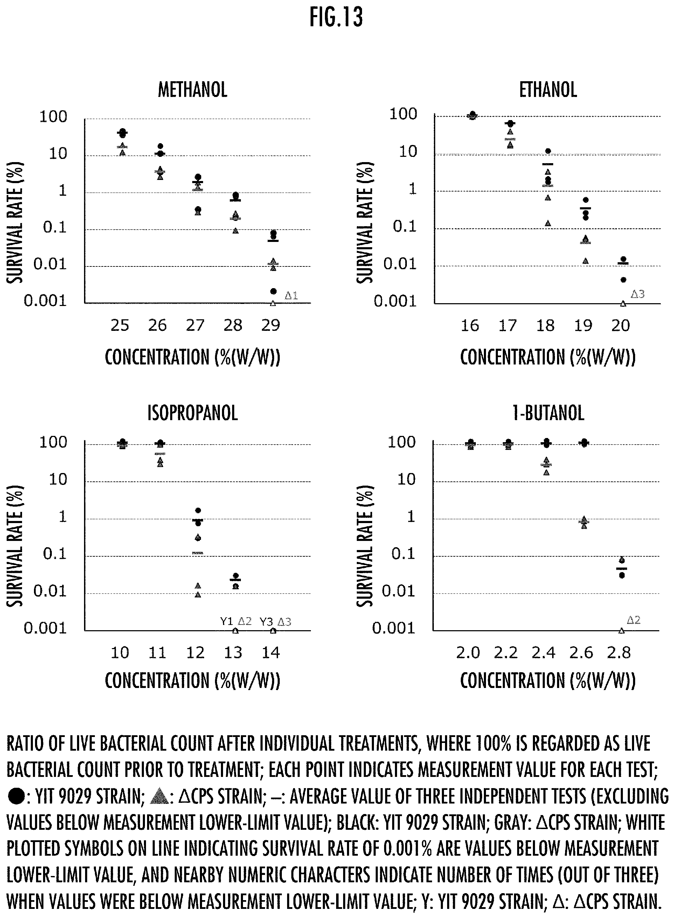

FIG. 13 is a set of graphs showing results from investigating the alcohol tolerance of the YIT 9029 strain and the Δcps strain in test example 8;

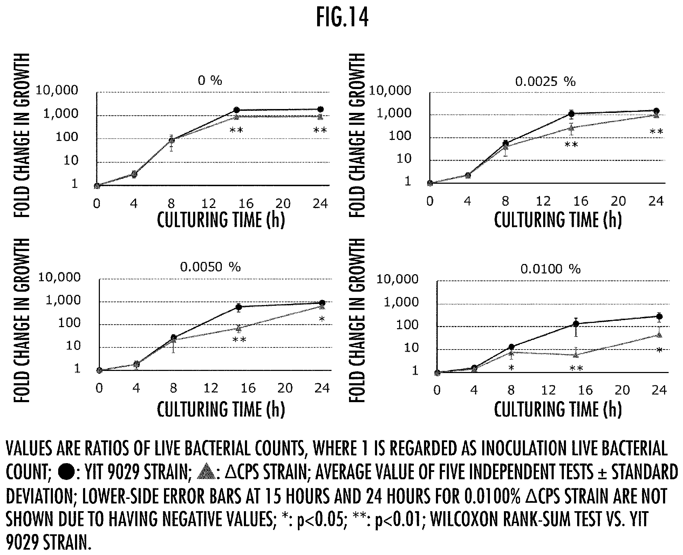

FIG. 14 is a set of graphs showing results from investigating the metal ion tolerance of the YIT 9029 strain and the Δcps strain in test example 9;

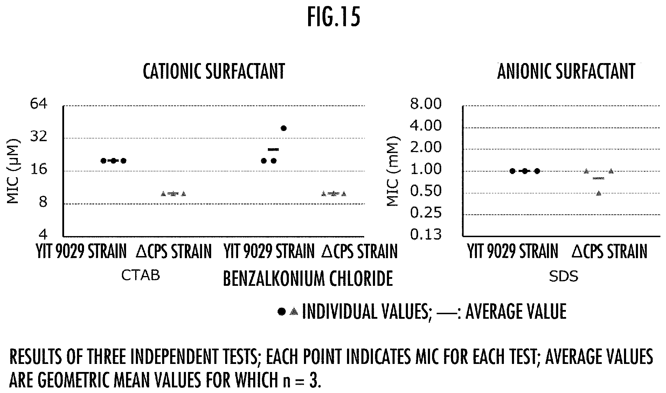

FIG. 15 is a set of graphs showing results from investigating the tolerance of the YIT 9029 strain and the Δcps strain to surface-active agents in test example 10;

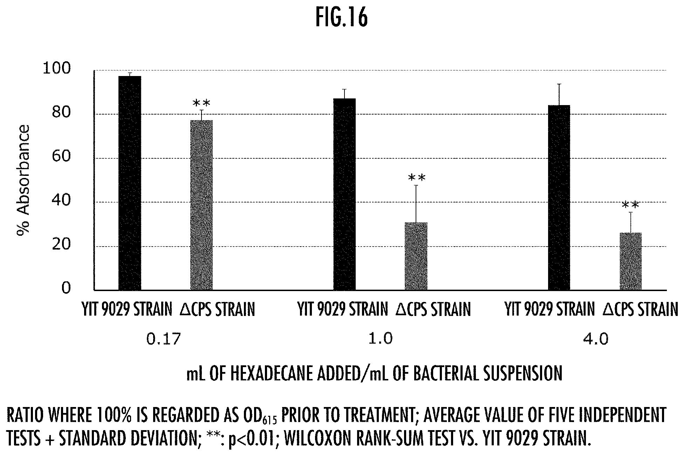

FIG. 16 is a bar graph showing results from investigating the adsorption of the YIT 9029 strain and the Δcps strain onto hexadecane in test example 11, said bar graph showing results obtained by recovering and measuring the turbidity (OD615) of the aqueous layer after the cell culture suspensions were treated with hexadecane;

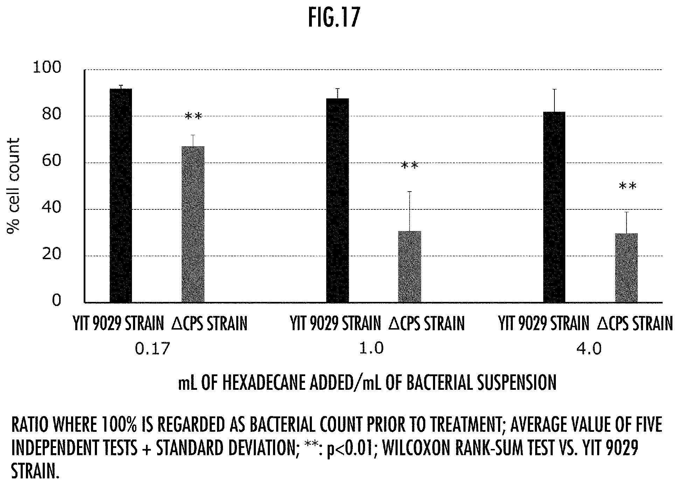

FIG. 17 is a bar graph showing results from investigating the adsorption of the YIT 9029 strain and the Δcps strain onto hexadecane in test example 11, said bar graph showing results obtained by recovering and measuring the number of bacteria contained in the aqueous layer after the cell culture suspensions were treated with hexadecane; and

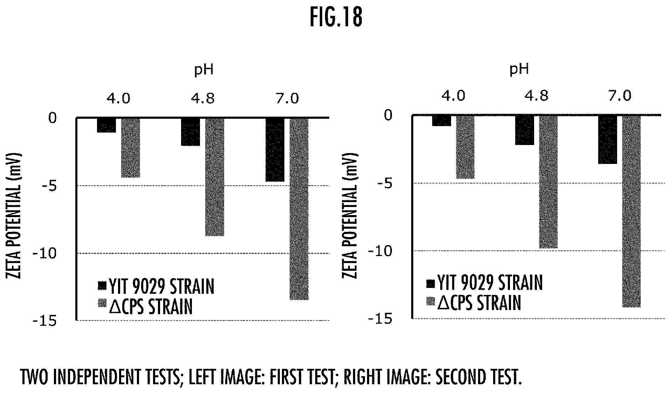

FIG. 18 is a set of bar graphs showing results from investigating the zeta potential of the YIT 9029 strain and the Δcps strain in test example 12.

### [MODE FOR CARRYING OUT THE INVENTION]

The composition according to the present invention is used for imparting environmental stress tolerance to bacteria.

In the present invention, "environmental stress tolerance" refers to a capability of bacteria to withstand environmental stress when exposed thereto, without undergoing any reduction in biological activity associated with mortality, growth capacity, gene expression, protein expression, enzyme activity, etc., or such that, despite a reduction in biological activity, the extent of such reduction is minimized. "Imparting" the aforementioned capability refers to providing said capability to bacteria; more specifically, when bacteria are treated with the composition according to the present invention, the biological activity of bacteria such as those described above is improved compared to that when the bacteria are not treated. In certain situations, it is possible to evaluate whether tolerance to environmental stress has been imparted to the bacteria by the composition according to the present invention by supplying bacteria treated with the composition according to the present invention and untreated control bacteria to a prescribed stressful environment and endeavoring to test the biological activity of the bacteria.

Examples of environmental stress against which tolerance is to be imparted to bacteria by the composition according to the present invention include environmental stresses caused by: gastric acid, bile, and other digestive fluids; metabolites produced in association with fermentation of the bacteria; bactericides; and bacteriostatic agents. Examples of physical substances that serve as sources of environmental stress for the bacteria include acids, bile components, alcohols, metal ions, hydrophobic substances, and surface-active agents.

The acids that serve as sources of environmental stress are not particularly limited. Examples thereof include: hydrochloric acid, sulfuric acid, nitric acid, boric acid, phosphoric acid, and other inorganic acids; and lactic acid, acetic acid, formic acid, citric acid, oxalic acid, and other organic acids.

The bile components that serve as sources of environmental stress are not particularly limited. Examples thereof include cholic acid, glycocholic acid, taurocholic acid, chenodeoxycholic acid, glycochenodeoxycholic acid, taurochenodeoxycholic acid, deoxycholic acid, glycodeoxycholic acid, taurodeoxycholic acid, and bile.

The alcohols that serve as sources of environmental stress are not particularly limited. Examples thereof include: methanol, ethanol, 1-propanol, isopropanol (2-propanol), 1-butanol, 2-butanol, glycerol, and other lower alcohols; and C6 or greater higher alcohols.

The metal ions that serve as sources of environmental stress are not particularly limited. Examples thereof include copper ions, silver ions, zinc ions, cobalt ions, and nickel ions.

The hydrophobic substances that serve as sources of environmental stress are not particularly limited. Examples thereof include hexane, octane, decane, dodecane, tetradecane, hexadecane, benzene, phenol, toluene, benzoic acid, nitrobenzene, styrene, xylene, cresol, hydroquinone, acetone, chloroform, and diethyl ether. Low-polarity hydrophobic substances are more preferred.

The surface-active agents that serve as sources of environmental stress are not particularly limited. Examples thereof include hexadecyltrimethylammonium bromide (CTAB), benzalkonium chloride, benzethonium chloride, cetylpyridinium chloride, sodium lauryl sulfate (SDS), alkyldiaminoethylglycine chloride, bile acids, and fatty acids. Cationic surface-active agents are more preferred.

The composition according to the present invention includes a cps1 gene cluster derived from *Lacticaseibacillus paracasei.* The cps1 gene cluster is introduced into designated bacteria, and tolerance to environmental stress is imparted to the bacteria. Thus, in another sense, the present invention provides a method for imparting environmental stress tolerance to bacteria, the method including a step in which, using the aforementioned composition, the cps1 gene cluster is introduced into designated bacteria. The cps1 gene cluster is, more specifically, a gene cluster including a cps1A gene, a cps1B gene, a cps1C gene, a cps1D gene, a cps1E gene, a cps1F gene, a cps1G gene, a cps1H gene, a cps1I gene, and a cps1J gene. The DNA sequence for the aforementioned cps1 gene cluster is registered as accession no. AB470649.1 in the GenBank of the National Center for Biotechnology Information (NCBI) and can be used by persons skilled in the art. The aforementioned gene cluster used in the present invention may be referred to simply as "the cps1 gene cluster" below.

The cps1A gene may have the polynucleotide sequence indicated by SEQ ID NO: 1 or a polynucleotide sequence that is at least 90% homologous thereto. Additionally, the cps1A gene may code the amino acid sequence indicated by SEQ ID NO: 11 or an amino acid sequence that is at least 90% homologous thereto. The homology is preferably 90% or higher, 96% or higher, 97% or higher, 98% or higher, or 99% or higher because the cps1A gene functionality can be demonstrated if the homology is at or above a given level.

The cps1B gene may have the polynucleotide sequence indicated by SEQ ID NO: 2 or a polynucleotide sequence that is at least 90% homologous thereto. Additionally, the cps1B gene may code the amino acid sequence indicated by SEQ ID NO: 12 or an amino acid sequence that is at least 90% homologous thereto. The homology is preferably 90% or higher, 96% or higher, 97% or higher, 98% or higher, or 99% or higher because the cps1B gene functionality can be demonstrated if the homology is at or above a given level.

The cps1C gene may have the polynucleotide sequence indicated by SEQ ID NO: 3 or a polynucleotide sequence that is at least 90% homologous thereto. Additionally, the cps1C gene may code the amino acid sequence indicated by SEQ ID NO: 13 or an amino acid sequence that is at least 90% homologous thereto. The homology is preferably 90% or higher, 96% or higher, 97% or higher, 98% or higher, or 99% or higher because the cps1C gene functionality can be demonstrated if the homology is at or above a given level.

The cps1D gene may have the polynucleotide sequence indicated by SEQ ID NO: 4 or a polynucleotide sequence that is at least 90% homologous thereto. Additionally, the cps1D gene may code the amino acid sequence indicated by SEQ ID NO: 14 or an amino acid sequence that is at least 90% homologous thereto. The homology is preferably 90% or higher, 96% or higher, 97% or higher, 98% or higher, or 99% or higher because the cps1D gene functionality can be demonstrated if the homology is at or above a given level.

The cps1E gene may have the polynucleotide sequence indicated by SEQ ID NO: 5 or a polynucleotide sequence that is at least 90% homologous thereto. Additionally, the cps1E gene may code the amino acid sequence indicated by SEQ ID NO: 15 or an amino acid sequence that is at least 90% homologous thereto. The homology is preferably 90% or higher, 96% or higher, 97% or higher, 98% or higher, or 99% or higher because the cps1E gene functionality can be demonstrated if the homology is at or above a given level.

The cps1F gene may have the polynucleotide sequence indicated by SEQ ID NO: 6 or a polynucleotide sequence that is at least 90% homologous thereto. Additionally, the cps1F gene may code the amino acid sequence indicated by SEQ ID NO: 16 or an amino acid sequence that is at least 90% homologous thereto. The homology is preferably 90% or higher, 96% or higher, 97% or higher, 98% or higher, or 99% or higher because the cps1F gene functionality can be demonstrated if the homology is at or above a given level.

The cps1G gene may have the polynucleotide sequence indicated by SEQ ID NO: 7 or a polynucleotide sequence that is at least 90% homologous thereto. Additionally, the cps1G gene may code the amino acid sequence indicated by SEQ ID NO: 17 or an amino acid sequence that is at least 90% homologous thereto. The homology is preferably 90% or higher, 96% or higher, 97% or higher, 98% or higher, or 99% or higher because the cps1G gene functionality can be demonstrated if the homology is at or above a given level.

The cps1H gene may have the polynucleotide sequence indicated by SEQ ID NO: 8 or a polynucleotide sequence that is at least 90% homologous thereto. Additionally, the cps1H gene may code the amino acid sequence indicated by SEQ ID NO: 18 or an amino acid sequence that is at least 90% homologous thereto. The homology is preferably 90% or higher, 96% or higher, 97% or higher, 98% or higher, or 99% or higher because the cps1H gene functionality can be demonstrated if the homology is at or above a given level.

The cps1I gene may have the polynucleotide sequence indicated by SEQ ID NO: 9 or a polynucleotide sequence that is at least 90% homologous thereto. Additionally, the cps1I gene may code the amino acid sequence indicated by SEQ ID NO: 19 or an amino acid sequence that is at least 90% homologous thereto. The homology is preferably 90% or higher, 96% or higher, 97% or higher, 98% or higher, or 99% or higher because the cps1I gene functionality can be demonstrated if the homology is at or above a given level.

The cps1J gene may have the polynucleotide sequence indicated by SEQ ID NO: 10 or a polynucleotide sequence that is at least 90% homologous thereto. Additionally, the cps1J gene may code the amino acid sequence indicated by SEQ ID NO: 20 or an amino acid sequence that is at least 90% homologous thereto. The homology is preferably 90% or higher, 96% or higher, 97% or higher, 98% or higher, or 99% or higher because the cps1J gene functionality can be demonstrated if the homology is at or above a given level.

In the cps1 gene cluster, or the cps1A gene, the cps1B gene, the cps1C gene, the cps1D gene, the cps1E gene, the cps1F gene, the cps1G gene, the cps1H gene, the cps1I gene, and the cps1J gene that are included therein, it is possible to synthesize DNA provided with the polynucleotide sequences thereof by using a means that is well known to persons skilled in the art, such as PCR or DNA synthesis. It is also possible to carry out cloning, amplification, or sequence confirmation using a typical cloning vector. Examples of cloning vectors include pUC18, pUC19, pBluescript II, T-Vector pMD20, and pYSSE3.

The cps1 gene cluster, or the cps1A gene, the cps1B gene, the cps1C gene, the cps1D gene, the cps1E gene, the cps1F gene, the cps1G gene, the cps1H gene, the cps1I gene, and the cps1J gene that are included therein, can be introduced into bacterial cells using a gene introduction means that is well known to persons skilled in the art. Examples of such means include chemical transformation, electroporation, viral vectors, and Crispr/Cas9. The means is not limited; according to a given aspect, because there are known to be external gene introduction vectors that are suitable for specific bacterial species, DNA that includes the gene sequence is preferably inserted into such a vector, and then the vector is preferably introduced into the bacterial cells. The vector can be introduced into the bacterial cells using, e.g., electroporation. The external gene introduction vectors are not limited; examples thereof include pLP10, pYAP300, and pYAP310.

The composition according to the present invention is preferably configured to contain the cps1 gene cluster, or the cps1A gene, the cps1B gene, the cps1C gene, the cps1D gene, the cps1E gene, the cps1F gene, the cps1G gene, the cps1H gene, the cps1I gene, and the cps1J gene that are included therein, in the form of the external gene introduction vector when said gene cluster or genes are incorporated in said vector as described above, but the form of the composition is not particularly limited. For example, the composition may be in solid form, liquid form, or another form.

In the present invention, the designated bacteria to which tolerance to environmental stress is to be imparted are not particularly limited; examples thereof include lactic acid bacteria. The species of lactic acid bacteria are not particularly limited; examples thereof include: *Lacticaseibacillus paracasei, Lacticaseibacillus casei,* and other bacteria of the genus *Lacticaseibacillus; Lactobacillus gasseri, Lactobacillus acidophilus, Lactobacillus cremoris, Lactobacillus helveticus, Lactobacillus delbrueckii* subsp. *bulgaricus, Lactobacillus delbrueckii* subsp. *delbrueckii, Lactobacillus johnsonii,* and other bacteria of the genus *Lactobacillus; Ligilactobacillus salivarius* and other bacteria of the genus *Ligilactobacillus; Limosilactobacillus fermentum* and other bacteria of the genus *Limosilactobacillus; Liquorilactobacillus mali* and other bacteria of the genus *Liquorilactobacillus; Lactiplantibacillus plantarum* and other bacteria of the genus *Lactiplantibacillus; Streptococcus thermophilus* and other bacteria of the genus *Streptococcus; Lactococcus lactis* subsp. *lactis, Lactococcus lactis* subsp. *cremoris, Lactococcus plantarum, Lactococcus raffinolactis,* and other bacteria of the genus *Lactococcus; Enterococcus faecalis, Enterococcus faecium,* and other bacteria of the genus *Enterococcus;* and *Bifidobacterium breve, Bifidobacterium bifidum, Bifidobacterium longum, Bifidobacterium animalis, Bifidobacterium suis, Bifidobacterium infantis, Bifidobacterium adolescentis, Bifidobacterium catenulatum, Bifidobacterium pseudocatenulatum, Bifidobacterium lactis, Bifidobacterium globosum,* and other bacteria of the genus *Bifidobacterium.* In the present description, "lactic acid bacteria" generally includes bacteria of the type referred to as Bifidobacteria. One type of lactic acid bacteria may be used alone, or two or more types of lactic acid bacteria may be used in combination.

In recent years, lactic acid bacteria of the genus *Lactobacillus* have been reclassified. Specifically, the bacterial genus of lactic acid bacteria belonging to the genus *Lactobacillus* in the past has been subdivided, and the names of some of the bacterial species have been updated.

### (Reclassification of lactic acid bacteria)

- Zheng et al. A taxonomic note on the genus Lactobacillus: Description of 23 novel genera, emended description of the genus Lactobacillus Beijerinck 1901, and union of Lactobacillus and Leuconostocaceae. Int. J. Syst. Evol. Microbiol. 2020 Apr; 70(4):2782-2858 DOI 10.1099/ijsem.0.004107.

Accordingly, in the present description, such lactic acid bacteria are indicated using the notation of the new classifications from after the reclassification. For example, among lactic acid bacteria that were classified as *Lactobacillus casei* or *Lactobacillus paracasei* under the old classification, those that can be newly classified as *Lacticaseibacillus paracasei* are included in the *Lacticaseibacillus paracasei* of the present application.

For bacteria into which the cps1 gene cluster is introduced, or into which the cps1A gene, the cps1B gene, the cps1C gene, the cps1D gene, the cps1E gene, the cps1F gene, the cps1G gene, the cps1H gene, the cps1I gene, and the cps1J gene that are included therein are introduced, appropriate and ideal culture media, culturing methods, preservation methods, etc., are known in accordance with the type of the bacteria. Therefore, bacteria into which the genes have been introduced (transformants) can be propagated or maintained in accordance with well-known methods that are thus suitable for the bacteria. Examples of preferred culture media for *Lacticaseibacillus paracasei,* which is a type of lactic acid bacteria, include Rogosa culture medium (Rogosa et al. A Selective Medium for the Isolation and Enumeration of Oral Lactobacilli. Journal of Dental Research, 1951 Oct; 30(5): 682-689. doi:10.1177/00220345510300051201), de Man Rogosa Sharpe (MRS) culture medium (de Man et al. A Medium for the Cultivation of Lactobacilli. Journal of Applied Bacteriology, 1960 Apr; 23(1): 130-135. doi:10.1111/j.1365-2672.1960.tb00188.x), milk, skim milk, soy milk, fruit juices, and vegetable juices. Examples of commercially available products include "Difco Lactobacilli MRS broth" (Becton, Dickinson and Co.), "GAM bouillon" (Nissui Pharmaceutical Co., Ltd.), "Modified GAM bouillon" (Nissui Pharmaceutical Co., Ltd.), and "MRS broth" (Merck KGaA). Culturing can include inoculating the bacteria into a liquid culture medium and carrying out agitation culturing or stationary culturing at 30 to 42°C, or applying the bacteria to a plate culture medium and carrying out stationary culturing under the same temperature conditions. After the culturing using the liquid culture medium, a culture supernatant is removed through centrifugal separation or the like, whereby it is possible to collect the bacteria. Additionally, fresh water or an aqueous buffer solution can be added to the collected bacteria to form a suspension, and the supernatant can be removed through further centrifugal separation or the like, to wash the bacteria.

The bacteria into which the genes have been introduced according to the present invention (transformants) can be used in various food ingredients, formulation ingredients, or the like, or in foods/beverages, medicines, supplements, or the like as appropriate, in accordance with various desired demands. The bacteria can be prepared in such forms by using a means that is well known in the manufacture of foods/beverages or a means that is well known in the formulation of medicines or supplements.

### [Examples]

The present invention is described more specifically below by way of examples, but these examples in no way limit the scope of the present invention.

### [1. Materials and methods]

### (1) Bacterial strains provided for testing

### <1> YIT 9029 strain

*Lacticaseibacillus paracasei* YIT 9029 strain. Internationally deposited as *Lactobacillus casei* YIT 9029 (FERM BP-1366; entrustment date: 1 May 1981) in the NITE Biological Resource Center (room 120, 2-chome-5-8 Kazusakamatari, Kisarazu, Chiba, Japan 292-0818), which is an International Depositary Authority under the Budapest Treaty. This bacterial strain may be referred to as "the YIT 9029 strain" below.

### <2> Development of Δcps strain

A region measuring approximately 11 kb in total length is present in the genome of *Lacticaseibacillus paracasei* YIT 9029, said region being known as a cell-wall-associated polysaccharide synthesis (cps) gene cluster (may be referred to as "the cps1 gene cluster" below) (Appl. Environ. Microbiol. (2008), 74(15), pp. 4746-4755). Table 1 shows the ten genes included in the cps1 gene cluster, the length of amino acid sequences of gene products thereof, and functions predicted from the amino acid sequences.

Using the YIT 9029 strain as a host, a bacterial strain was constructed in which a total length of approximately 11 kb of the cell-wall-associated polysaccharide synthesis gene cluster (cps1 gene cluster) of the host was completely deleted through double crossover. This bacterial strain may be referred to as "the Δcps strain" below.

**[Table 1]**

| Table 1: cps1 gene cluster | | | | |
|---|---|---|---|---|
| Gene | SEQ ID NO (cDNA sequence) | Amino acid sequence length | SEQ ID NO (amino acid sequence) | Predicted function |
| cps1A | SEQ ID NO: 1 | 309 a.a. | SEQ ID NO: 11 | Chain length determination |
| cps1B | SEQ ID NO: 2 | 252 a.a. | SEQ ID NO: 12 | Capsular polysaccharide biosynthesis |
| cps1C | SEQ ID NO: 3 | 393 a.a. | SEQ ID NO: 13 | Rhamnosyltransferase |
| cps1D | SEQ ID NO: 4 | 385 a.a. | SEQ ID NO: 14 | Glycosyltransferase |
| cps1E | SEQ ID NO: 5 | 324 a.a. | SEQ ID NO: 15 | Glycosyltransferase |
| cps1F | SEQ ID NO: 6 | 419 a.a. | SEQ ID NO: 16 | - |
| cps1G | SEQ ID NO: 7 | 221 a.a. | SEQ ID NO: 17 | Galactoside acetyltransferase |
| cps1H | SEQ ID NO: 8 | 478 a.a. | SEQ ID NO: 18 | Polysaccharide repeat unit transporter |
| cps1I | SEQ ID NO: 9 | 295 a.a. | SEQ ID NO: 19 | Glycosyltransferase |
| cps1J | SEQ ID NO: 10 | 238 a.a. | SEQ ID NO: 20 | Sugar transferase |

### <2.1> Production of insertion fragment

From the genome extracted from the YIT 9029 strain, individual DNA fragments were amplified through PCR by using primers indicated by SEQ ID NOs: 21 and 22 for the DNA sequence measuring approximately 1 kb in length upstream of the cps1A gene and using primers indicated by SEQ ID NOs: 23 and 24 for the DNA sequence measuring approximately 1 kb in length downstream of the cps1J gene. In this instance, primers were designed such that a sequence equivalent to 18 bases at both ends produced when a plasmid vector pYSSE3 (which shall be described later) configured in linear form was added to one terminal of each of two types of resulting amplification products (see SEQ ID NOs: 22 and 23). Concurrently with the foregoing step, primers were used, where each primer was designed such that an overlapping region of 16-23 bases in length at the ends of both sequences was added to the other terminal of each of the two types of resulting amplification products (see SEQ ID NOs: 21 and 24). Next, the two sequences were joined through PCR by using the overlap region (using the primers indicated by SEQ ID NOs: 23 and 22), and an insertion fragment measuring approximately 2k bases in total length was obtained. It was confirmed, through PCR using primers indicated by SEQ ID NOs: 25 and 26 for amplifying 250 bp preceding and following the junction, that joining was reliably carried out.

### <2.2> Production and selection of plasmid including insertion fragment

pYSSE3 was used in cloning the insertion fragment developed as a PCR amplification product. pYSSE3 is a gene-cloning plasmid vector provided with a duplication gene present in *Escherichia coli,* the plasmid vector being provided with an erythromycin resistance gene that functions as a growth selection marker in both *Escherichia coli* and lactic acid bacteria. The PCR amplification product was joined, through seamless cloning using an "In-Fusion HD Cloning Kit" (Takara Bio Inc.), to the pYSSE3 configured in linear form through PCR.

The insertion fragment was confirmed through colony PCR using primers indicated by SEQ ID NOs: 27 and 28, the primers being derived from the pYSSE3 vector sequence. The sequence of the insertion fragment was sequenced using the primers as sequencing primers indicated by SEQ ID NOs: 25 to 28, and it was confirmed that there were no errors in the sequence.

### <2.3> Introduction of plasmid into YIT 9029 strain and selection of recombinant

The stationary-phase YIT 9029 strain was inoculated in an amount of 5% (v/v) in 20 mL of a liquid culture medium, specifically "Difco Lactobacilli MRS broth" (Becton, Dickinson and Co.) (may be referred to as "MRS" below), which is a culture medium for lactic-acid-bacteria, and the inoculated bacteria were cultured for two hours at 37°C. After 15 minutes of being cooled with ice, the bacteria were centrifuged for 5 minutes at 7,000 rpm and washed with 20 mL of 1 mM HEPES (pH 7). The bacteria were centrifuged again and then washed with 10 mL of 10% (v/v) glycerol. After being centrifuged yet again, the bacteria were suspended in 200 µL of 10% (v/v) glycerol, transferred to a 1.5-mL tube, and centrifuged for 5 minutes at 15,000 rpm. The bacteria were suspended in 100 µL of 10% (v/v) glycerol, after which 1 µL of a constructed plasmid was added to 40 µL of the suspension, and the resulting combination was mixed through pipetting. The resulting mixture was added to a 2-mm-wide electroporation cuvette, and pulses were applied to the mixture at 25 µF, 200 Ω, and 1.5 KV using a "Gene Pulser II" (Bio Rad Laboratories, Inc.), which is an electroporation device. The pulse-treated mixture was suspended in 1 mL of an MRS liquid medium and cultured for 1 hour at 37°C. Subsequently, 200 µL of the mixture (per plate) was spread onto the surface of an MRS agar medium containing 20 µg/mL of erythromycin using a cell spreader. After the plates were cultured for 3 days at 37°C, some of the resulting colonies were supplied for testing in colony PCR, and it was confirmed whether the plasmid had integrated into the genome at the target locus. Colonies in which integration of the plasmid into the genome was confirmed were collected and cultured for 24 hours at 37°C using an MRS liquid medium containing 20 µg/mL of erythromycin.

### <2.4> Acquisition of gene-disrupted strain and confirmation of gene disruption

Integration of the introduced plasmid into the genome was confirmed through PCR using the primer indicated by SEQ ID NO: 28 derived from the sequence of the pYSSE3 vector and a primer (SEQ ID NO: 29) derived from the sequence downstream of the cps1 gene cluster, which should be deleted in the targeted gene-disrupted Δcps strain, or in the same manner, through PCR using the primer indicated by SEQ ID NO: 27 derived from the sequence of the pYSSE3 vector and a primer (SEQ ID NO: 30) derived from the sequence upstream of the cps1 gene cluster which should be deleted in the targeted mutant. Bacterial strains for which the insertion could be confirmed were subcultured twice using an MRS liquid medium containing no erythromycin. The liquid culture was spread onto a modified Rogosa plate medium, and grown colonies that were irregularly shaped relative to the wild type strain were selected and subjected to colony PCR using the primers indicated by SEQ ID NOs: 29 and 30, and it was confirmed that the entirety of the cps1 gene cluster was deleted and that there was no further deletion in the same gene region.

**[Table 2]**

| Table 2: Primer sequences | | | |
|---|---|---|---|
| Description of amplified/extended sequence | Primer orientation | Sequence (5' → 3') | SEQ ID NO |
| YIT 9029 strain genome: | Forward | 5'-CACTGCGAAAAAAATCTAACTCTTTCCCCACCTAATC-3' | SEQ ID NO: 21 |
| amplification of approximately 1 kb upstream of cps1A and joining of amplification product from approximately 1 kb downstream of cpslJ | | | |
| | Reverse | 5'-CTGCAGGTCGACTCTAGAGCAATCAACGTGTAGTAATGC-3' | SEQ ID NO: 22 |
| YIT 9029 strain genome: | Forward | 5'-ACCCGGGGATCCTCTAGAGACCATAATCATTCTTTTTGAGTG- 3' | SEQ ID NO: 23 |
| amplification of approximately 1 kb downstream of cps1J and joining of amplification product from approximately 1 kb upstream of cps1A | | | |
| | Reverse | 5'- GGGATTAGGTGGGGAAAGAGTTAGATTTTTTTCGCAGTGTAAAAAG- 3' | SEQ ID NO: 24 |
| Amplification of 250 b preceding and following junction | Forward | 5'-TTTGATATAGACTCCGTTATCGG-3' | SEQ ID NO: 25 |
| | Reverse | 5'-TCATTCTGACGGGTTTATAGG-3' | SEQ ID NO: 26 |
| pYSSE3-derived sequence | Forward | 5'-TTAAGTTGGGTAACGCCAGG-3' | SEQ ID NO: 27 |
| | Reverse | 5'-GGATAACAATTTCACACAGGA-3' | SEQ ID NO: 28 |
| Sequences upstream and downstream of cps1 gene cluster deleted in Δcps strain | Forward | 5'-CGATTGTCACCAAAAACATC-3' | SEQ ID NO: 29 |
| | Reverse | 5'-GGAAGGGATACACCCTAAATC-3' | SEQ ID NO: 30 |

### (2) Culturing and washing of bacteria

The bacterial strains were cultured in MRS liquid medium for 24 hours to stationary phase, after which a 0.1% (v/v) aliquot of the culture was inoculated into 10 to 30 mL of fresh MRS liquid medium and cultured at 37°C for 24 hours. The resulting bacterial culture was centrifuged for 15 minutes at 10,000 × g, and the supernatant was removed. Pure water in an amount equivalent to that of the original liquid culture was added to the precipitate, and the bacteria were suspended through pipetting. The cycle of centrifugation and suspension was repeated once under the same conditions, and the precipitate resulting after further centrifugation was regarded as washed cells.

### (3) Preparation of whole-cell-wall polysaccharide solution

A polysaccharide extract solution obtained by extracting cell-wall polysaccharides from the lactic acid bacteria was prepared in the manner described below. First, L-arabinose (Fujifilm Wako Pure Chemical Corp.) was dissolved in a 5 mM tris-maleate buffer (pH 7.0) so that the concentration was 1.5 M. The aforementioned washed cells were suspended in the resulting solution and treated with a cell-wall-lytic enzyme. Specifically, 100 µg of "Lysozyme from chicken egg white lyophilized powder" (Sigma-Aldritch Co.) and 10 µL (100 U) of "Mutanolysin (recombinant)" (A&A Biotechnology) were added per milliliter of the suspension. The resulting suspension was treated at 50°C for 2 hours or more until the suspension turned transparent. After the treatment, the cell debris was removed by centrifuging the suspension for 15 minutes at 10,000 × g, and by passing the resulting supernatant through a "33-mm Millex-GP" (Merck KGaA), which is a 0.22-µm sterilization filter. The resulting solution was added into an "Amicon Ultra-15 10 kDa MWCO" (Merck KGaA) (nominal-fraction molecular weight: 10,000) centrifugal ultrafiltration unit, and subjected to centrifugal filtration for 30 minutes at 5,000 × g. A cycle of adding 11 mL of sterilized water to the resulting retentate and carrying out centrifugal filtration for 30 minutes at 5,000 × g was repeated twice. Approximately 1 mL of the resulting retentate was recovered, and 9 mL of a 5.5 mM tris-maleate buffer having a pH of 7.0 (containing no sugars) was added thereto, whereupon the resulting combination was treated with a protease and a nuclease. Specifically, 20 µg of "Pronase Protease, *Streptomyces griseus"* (Merck KGaA), which is a protease formulation, and 20 µg of trypsin (Sigma-Aldritch Co.) were added per milliliter of the solution, and the resulting combination was treated for 24 hours at 37°C. The resulting reaction solution was treated for 10 minutes at 100°C and enzymatically deactivated, after which 1 µL of "Benzonase Nuclease" (Merck KGaA) was added per milliliter of the solution, and the resulting combination was treated for 24 hours at 37°C. The resulting reaction solution was treated for 10 minutes at 100°C and enzymatically deactivated, after which the reaction solution was added into an "Amicon Ultra-15 10 kDa MWCO" (Merck KGaA) (nominal-fraction molecular weight: 10,000) centrifugal ultrafiltration unit, and subjected to centrifugal filtration for 30 minutes at 5,000 × g. A cycle of adding 11 mL of pure water to the resulting retentate and carrying out centrifugal filtering for 30 minutes at 5,000 × g was repeated twice. Approximately 1 mL of the retentate in total was added into a "Spectra/Por Dialysis Membrane MWCO = 6-8,000" (Spectrum Medical Industries) dialysis tube and dialyzed for 24 hours at room temperature. The resulting dialyzed solution within the tube was regarded as a whole-cell-wall polysaccharide solution.

### (4) Separation and analysis of polysaccharide-containing preparation through gel filtration chromatography

Separation and analysis were carried out using an "Alliance 2695 Separations Module" (Waters Corp.) HPLC system, under the conditions shown in table 3. The resulting chromatogram was analyzed using the analysis applications, "Empower" (Waters Corp.) or "Empower 3" (Waters Corp.). Pullulan P100, P200, P400, and P800 (Showa Denko KK), polyethylene oxide M. W. 1,000,000 (PEO; Thermo Fisher Scientific), and polyethylene glycol 2,000,000 (PEG; Fujifilm Wako Pure Chemical Corp.) were used as reference standards. A calibration curve was created from the molecular weights of the reference standards (as specified in the manufacturer's documentations) and the elution time in HPLC, and the peak molecular weight of the sample was estimated.

**[Table 3]**

| Table 3: Separation conditions employed in gel filtration chromatography | |
|---|---|
| System | Alliance 2695 Separations Module HPLC system (Waters Corp.) |
| Column | Shodex OHpak SB-G + SB-806 HQ (diameter 6.0 mm × 50 mm + diameter 8.0 mm × 300 mm) |
| Mobile phase | 50 mM NaCl |
| Flow rate | 0.5 mL/min |
| Column temperature | 60°C |
| Detector | Waters 2414 Refractive Index Detector and Waters 2489 UV/Visible Detector |
| RI detector temperature | 30°C |

### [2. Tests and results]

### [Test example 1] (Observation under microscope)

MRS liquid medium cultures of the YIT 9029 strain and the Δcps strain were diluted 10-fold using pure water, and 1 µL of each diluted solution was applied to an "MAS-coated glass slide" (Matsunami Glass Ind., Ltd.) anti-peeling slide. After being air-dried, the slide was thermally fixed using a Bunsen burner and gram-stained. Specimens were subjected to bright-field observation using a "Leica DM5500 B" (Leica Biosystems, Inc.) optical microscope.

According to the results, when microscopic images of the YIT 9029 strain and the Δcps strain cultured using the MRS liquid medium were observed, autoaggregation of the cells was observed to a higher extent in the Δcps strain than in the YIT 9029 strain, as shown in FIG. 1.

### [Test example 2] (Antibody reactivity)

MRS liquid medium cultures of the YIT 9029 strain and the Δcps strain were diluted 10-fold using pure water and centrifuged for 5 minutes at 10,000 × g. Pellets were suspended in 0.01 M phosphate-buffered saline (PBS) and treated for 10 minutes at 100°C. After being centrifuged for 5 minutes at 10,000 × g, the pellets were suspended in PBS containing 2% (w/v) bovine serum albumin (BSA) and antibodies specific to the cell-wall polysaccharides, said antibodies being diluted 10-fold, and the suspension was subjected to reaction for 30 minutes at 37°C. After being centrifuged for 5 minutes at 10,000 × g, the pellets were suspended in PBS and centrifuged again for 5 minutes at 10,000 × g, after which the pellets were suspended in PBS containing 2% (w/v) BSA and "Alexa Fluor 555 goat anti-mouse IgM" (Thermo Fisher Scientific, Inc.) secondary antibodies diluted 500-fold, and the suspension was subjected to reaction for 30 minutes at 37°C. The operation for carrying out centrifugation for 5 minutes at 10,000 × g and then suspending the pellets in PBS was performed twice, and 1 µL of the resulting suspension was applied to each section of a 1 cm × 1 cm sectioned MAS-coated glass slide (Matsunami Glass Ind., Ltd.). After the slide was air-dried, 4.5 µL/section of "VECTASHIELD Mounting Medium for Fluorescence with DAPI H-1200" (Vector Laboratories, Inc.) fluorescence staining sealant, was added to the sections of the slide, and cover glass was placed on the slide from above. The specimens were subjected to phase contrast observation (×280), bright-field observation (×700), and fluorescence observation using a "Leica DM5500 B" (Leica Biosystems, Inc.) optical microscope, and digital images were acquired using a "Leica MMAF system" (Leica Biosystems, Inc.) imaging system. A fluorescence image was obtained by monochromatically imaging "Alexa Fluor 555" (excitation light: 555 nm; released fluorescence: 585 nm)-fluorescent secondary antibodies, subsequently converting the monochrome image to a green color in consideration of ease of visual confirmation, and overlaying the converted image onto the phase contrast image or the bright-field image in the "Leica MMAF system" imaging system.

According to the results, fluorescence produced by secondary antibodies was observed around each of the cells of the YIT 9029 strain, but no fluorescence was observed with the Δcps strain treated under the same conditions, as shown in FIG. 2.

### [Test example 3] (Separation and analysis of whole-cell-wall polysaccharide solution)

Sterilized water was added to the whole-cell-wall polysaccharide solution so that the volume was 10 mL, and the resulting combination was centrifugally filtered for 2 to 5 minutes at 5,000 × g using an "Amicon Ultra-15 100 kDa MWCO" (Merck KGaA) (nominal-fraction molecular weight: 100,000) centrifugal ultrafiltration unit, so that the volume was 1 mL or less. A cycle of adding 11 mL of pure water to the resulting retentate and carrying out centrifugal filtration for 2 to 5 minutes at 5,000 × g was furthermore repeated twice. The resulting retentate was regarded as a "100K retentate." In order to reduce the volume of the permeate (approximately 30 mL in total) obtained through centrifugal filtration steps, centrifugal filtration using the "Amicon Ultra-15 10 kDa MWCO" (nominal-fraction molecular weight: 10,000) centrifugal ultrafiltration unit was repeated at 5,000 × g until the volume was approximately 1 mL, and the resulting retentate was regarded as a "100K permeate."

FIG. 3 shows results from when the whole-cell-wall polysaccharide solution prior to ultrafiltration, the 100K retentate after ultrafiltration, and the 100K permeate after ultrafiltration were each separated and analyzed through gel filtration column chromatography.

As shown in FIG. 3, it was apparent that when the whole-cell-wall polysaccharide solution of the YIT 9029 strain was fractionated using an ultrafiltration filter having a nominal-fraction molecular weight (NMWL) of 100,000 (100K) (may be referred to as "the 100K filter" below), a portion of the substances exhibiting a peak on the high-molecular-weight side passed through the 100K filter (see the chromatograms in the left-side sections of FIG. 3). Specifically, the 100K retentate was found to have a peak indicating an elution time in which a peak top is at 17 minutes on the chromatogram, and the 100K permeate was found to have a peak indicating an elution time in which a peak top is at 16 minutes on the chromatogram. According to the calibration curve produced using the reference standards, the elution time of 16 minutes could be said to correspond to the elution time of a substance having a molecular weight of approximately 1,800,000, and the elution time of 17 minutes could be said to correspond to the elution time of a substance having a molecular weight of approximately 1,000,000.

However, in the case of the Δcps strain, although the 100K retentate was found to have a peak having a peak top at 17 minutes on the chromatogram, no peak having a peak top at 16 minutes was detected in the chromatogram for the 100K permeate (see the chromatograms in the right-side sections of FIG. 3).

It follows from the above that the peaks of substances configured to have different characteristics overlapped on the peak in the high-molecular-weight side observed when the whole-cell-wall polysaccharide solution of the YIT 9029 strain was separated and analyzed through gel filtration column chromatography. Additionally, because this phenomenon was not observed with the Δcps strain, the substance that passed through the 100K filter may have been cell-wall polysaccharides formed by the cps1 gene cluster. Additionally, since the substances passed through the 100K filter, the substances were presumed to be polysaccharides having a linear structure with few branches or with branches composed of short sugar chains.

### [Test example 4] (Evaluation of acid tolerance, part 1)

The Δcps strain, which is unable to form ordinary cell-wall polysaccharides due to the deletion of the cps1 gene cluster, was evaluated for tolerance to acids, and the YIT 9029 strain, the parent strain having ordinary cell-wall polysaccharides, was used as the control.

Specifically, a test was conducted in the manner described below. Specifically, liquid cultures of the YIT 9029 strain or the Δcps strain were prepared with the MRS liquid medium, and 90.9 µL of each liquid culture was added to 9 mL of 0.2 M glycine-hydrochloric acid buffer solutions (37°C) adjusted to various pH levels, and the components were mixed using a vortex mixer, after which the resulting mixture was allowed to stand at 37°C. 1 mL of each of the resulting treatment solutions was taken after predetermined time periods had elapsed and added to 0.01 M PBS (pH: 7.2 to 7.4), and then test samples that were diluted using PBS as appropriate were prepared and applied to an MRS plate medium. After the agar plate medium to which the test samples were applied were cultured for 2 days or longer at 37°C under aerobic conditions, the number of the colonies formed was measured, and the count of colony-forming units (CFUs) per milliliter of the bacterial culture was calculated from the dilution factor. Regarding the CFU counts as the live bacterial count, the survival rate was calculated from the live bacterial count of the liquid culture before and after various treatment lengths. For the treatment using a 0.2 M glycine aqueous solution (pH: 6.2) of which pH was not adjusted, the survival rate of both the YIT 9029 strain and the Δcps strain did not decrease at least until the 4th hour after the start of treatment (both survival rates were about 100%). Note that the change in pH of the treatment solutions with various pH adjustments was less than 0.1 with the start of the treatments in all cases.

FIG. 4 shows the average survival rate with standard deviation for five independent tests.

As shown in FIG. 4, in cases where the cells were treated with a glycine-hydrochloric acid buffer solution adjusted to a pH of 2.6 to 3.0 using hydrochloric acid, there was a significant reduction in survival rate for the Δcps strain in comparison with the YIT 9029 strain. In particular, with the treatments adjusted to a pH of 2.8 and those adjusted to a pH of 3.0, the live bacterial count for the Δcps strain was reduced by 30% or more even when the reduction in the live bacterial count for the YIT 9029 strain was less than 1% (from the start of treatment to after 60 minutes had elapsed).

### [Test example 5] (Evaluation of acid tolerance, part 2)

Growth under low pH conditions was evaluated for the Δcps strain, which is unable to form ordinary cell-wall polysaccharides due to the deletion of the cps1 gene cluster, with the parent YIT 9029 strain with ordinary cell-wall polysaccharides as the control.

Specifically, a test was conducted in the manner described below. Specifically, the YIT 9029 strain or Δcps strain cultured for 24 hours in an MRS liquid medium until reaching their stationary phase was inoculated into another fresh MRS liquid medium with its pH adjusted to 4.5 to 4.7 using hydrochloric acid, lactic acid, or acetic acid, or into MRS without pH adjustments (pH: 6.2 to 6.3), and then cultured for 3 to 24 hours at 37°C. After predetermined time periods had elapsed, aliquots of the liquid culture were taken, the live bacterial count was measured in the same manner as in test example 4, and the fold changes in growth were calculated using the bacterial count at inoculation as the denominator.

The results are shown in FIGS. 5 to 8.

FIG. 5 shows results of culturing the strains in a low-pH culture medium to which hydrochloric acid was added. Moreover, FIG. 5 shows the average growth curve with the standard deviation of five independent culturing tests.

As shown in FIG. 5, no difference in growth was found between the YIT 9029 strain and the Δcps strain in the case of the culture medium without pH adjustment (pH: 6.3). However, in the case of the culture medium with its pH adjusted to 4.5 through addition of hydrochloric acid, the fold change in growth of the Δcps strain was significantly decreased at all measurement times after three hours of culturing. The ratio of the fold changes in growth between the bacterial strains (YIT 9029 strain/Δcps strain) was at a maximum of 2.2 at the 12-hour time point after culturing, and was 1.4 at the 24-hour time point.

FIGS. 6 and 7 show results of culturing the strains in a low-pH culture medium to which lactic acid was added. Moreover, FIG. 7 shows the average and standard deviation of five independent culturing tests.

As shown in FIG. 6, the growth curve (n = 1) in the case of the culture medium without pH adjustments (pH: 6.3) showed no apparent difference between the fold change in growth of YIT 9029 strain and the Δcps strain. However, in the case of the culture medium with its pH adjusted to 4.7 through addition of lactic acid, the fold change in growth of the Δcps strain was decreased at all measurement times after three hours of culturing. The ratio of the fold change in growth between the bacterial strains (YIT 9029 strain/Δcps strain) was at a maximum of 2.7 at the 15-hour time point after culturing, and it was 1.7 at the 24-hour time point.

As shown in FIG. 7, the comparison of the growth capacityat the 12-hour time point of culturing in the case of the culture medium without pH adjustments (pH: 6.3) (n = 5) showed substantially no difference in the fold changes in growth between the YIT 9029 strain and the Δcps strain, and the ratio of the fold changes in growth between the bacterial strains (YIT 9029 strain/ Δcps strain) was merely 1.2. However, in the case of the culture medium with its pH adjusted to 4.7 through addition of lactic acid, the ratio of fold changes in growth (YIT 9029 strain/Δcps strain) was 2.2, said result consistent with when hydrochloric acid was added to the culture medium in test example 4.

FIG. 8 shows results of culturing the strains using a low-pH culture medium to which acetic acid was added. Moreover, FIG. 8 shows the average and standard deviation of five independent culturing tests.

As shown in FIG. 8, the comparison of the growth capacity at the 12-hour time point of culturing in the case of the culture medium without pH adjustments (pH: 6.2) (n = 5) showed no significant difference in fold changes in growth between the YIT 9029 strain and the Δcps strain. However, in the case of the culture medium with its pH adjusted to 4.7 through addition of acetic acid, the fold change in growth of the Δcps strain was significantly decreased, and the ratio of the fold changes in growth between the bacterial strains (YIT 9029 strain/Δcps strain) was 1.6.

### [Test example 6] (Evaluation of bile tolerance)

Tolerance to bile was evaluated for the Δcps strain, which is unable to form ordinary cell-wall polysaccharides due to deletion of the cps1 gene cluster, using the parent YIT 9029 strain having ordinary cell-wall polysaccharides as the control.

Specifically, a test was conducted in the manner described below. Specifically, first, a predetermined amount of sodium deoxycholate (Sigma Aldritch) or bovine bile powder ("Difco Oxgall"; Becton, Dickinson and Co.) was added to phosphate-buffered saline (pH: approximately 7.3), and the solution was sterilized using a 0.22 µm filter.

The component concentration and pH of the treatment solutions are as indicated below.
- Deoxycholic acid: 0.1% (w/v), pH: 7.0
- Bovine bile (10% (w/v) powder equivalent): 0.02 to 0.8% (w/v), pH: 7.0

Separately, 100 µL of liquid cultures of the YIT 9029 strain or the Δcps strain that were produced using the MRS liquid medium were each added to 9.9 mL of treatment solutions that were kept at 37°C, and the components were mixed using a vortex mixer, after which the resulting mixture was allowed to stand at 37°C. After aliquots of the treatment solutions were taken after predetermined time periods had elapsed, the live bacteria count was measured in the same manner as in test example 4, and the survival rates were calculated from the live bacteria count in the liquid culture solution before and after various treatment times had elapsed.

The results are shown in FIGS. 9 to 11.

FIG. 9 shows results from when the influence of a treatment carried out for 5 minutes using deoxycholic acid was investigated. Moreover, FIG. 9 shows the average and standard deviation of the survival rates from five independent tests.

As shown in FIG. 9, the survival rate of the Δcps strain was significantly decreased in comparison with the YIT 9029 strain. Specifically, the average value of the survival rates was 8 times higher in the YIT 9029 strain than in the Δcps strain.

FIG. 10 shows results from when the influence of a treatment carried out for 60 minutes using bovine bile was investigated. Moreover, FIG. 10 shows the average and standard deviation of the survival rates from five independent tests.

As shown in FIG. 10, it was found that the survival rate of the Δcps strain tended to be lower than that of the YIT 9029 strain. In particular, the survival rate was significantly decreased at a bovine bile concentration of 0.2% (w/v).

FIG. 11 shows results from when details pertaining to the change over time in survival rate at various concentrations of bovine bile were investigated (n = 1).

As shown in FIGS. 11A to 11C, the survival rate abruptly decreased before 5 minutes had elapsed from the start of treatment irrespective of the bacterial strain and the bovine bile concentration, and then the survival rate gradually decreased until 60 minutes had elapsed. In this instance, the survival rate of the Δcps strain was lower than that of the YIT 9029 strain, and the difference was found to be approximately 2- to 6-fold.

### [Test example 7] (Evaluation of acid/bile tolerance)

Tolerance to acid/bile was evaluated for the Δcps strain, which is unable to form ordinary cell-wall polysaccharides due to deletion of the cps1 gene cluster, using the parent YIT 9029 strain having ordinary cell-wall polysaccharides as the control.

Specifically, a test was conducted in the manner described below. Specifically, first, a 0.2 M glycine-hydrochloric acid buffer solution having a pH of 3.0 that was prepared according to test example 4, or the phosphate-buffered saline (pH: 7.0) having a bovine bile concentration of 0.2% (w/v) that was prepared according to test example 6, was prepared. Separately, liquid cultures of the YIT 9029 strain or the Δcps strain were prepared using the MRS liquid medium, and 1 mL of each liquid culture was added to 9 mL of a 0.2 M glycine-hydrochloric acid buffer solution (pH: 3.0) that was kept at 37°C, and the components were mixed using a vortex mixer, after which the resulting mixture was allowed to stand at 37°C. Aliquots of the liquid cultures were taken after 60 minutes had elapsed, and the survival rates for the tested samples were calculated in the same manner as in test example 4. Next, in cases involving treatment with bovine bile, another 1 mL of the treatment solutions was sampled and added to 9 mL of a phosphate-buffered saline (pH: 7.0) having a bovine bile concentration of 0.2% (w/v) that was kept at 37°C, and the components were mixed using a vortex mixture, after which the resulting mixture was allowed to stand at 37°C. Aliquots of the treatment solutions were taken after 60 minutes had elapsed, and the survival rates for the tested samples were calculated in the same manner as in test example 4.

The results are shown in FIG. 12.

As shown in FIG. 12, the survival rate of only the Δcps strain had decreased due to treatment for 60 minutes with the 0.2 M glycine-hydrochloric acid buffer solution (pH: 3.0) prepared using hydrochloric acid. Furthermore, after a subsequent treatment was performed for 60 minutes with bovine bile having a concentration of 0.2% (w/v), whereas the survival rate of the YIT 9029 strain was 73%, the survival rate of the Δcps strain had decreased to 19%.

### [Test example 8] (Evaluation of alcohol tolerance)

### • Treatment of cell bodies with alcohol

Liquid cultures of the YIT 9029 strain or the Δcps strain were cultured for 12 hours to a logarithmic growth phase using the MRS liquid medium (approximately 8 × 10⁸ CFU/mL for both strains) were added in amounts of 100 µL to 9.9 mL of an aqueous solution containing an alcohol (methanol, ethanol, isopropanol, or 1-butanol) in various concentrations, and the resulting combinations were allowed to stand for 10 minutes at room temperature. The live bacterial counts were measured before and after treatment, and the survival rate was calculated as the ratio using the count of live bacteria added to the treatment as the denominator. The average survival rate of three independent tests was calculated. The limit of detection for the live bacterial count was 200 CFU/mL (survival rate: approximately 0.002%). Data that was below the limit of detection was indicated on the graph as a survival rate of 0.001% but was not included in the calculation of the average value.

### • Measurement of live bacterial count

The liquid cultures were diluted as appropriate using saline, after which the diluted bacterial suspension was spread onto an MRS plate medium produced in advance, using a spiral plater EDDY JET 2 (IUL Instruments GmbH). Colonies grown on a culture medium that was cultured for 2 days or longer at 37°C were counted using an automated colony counter ProtoCOL 3 (Synoptics Ltd.), and the resulting CFU counts were regarded as the live bacteria count.

### • Results

As shown in FIG. 13, it was found that the survival rate of the YIT 9029 strain tended to be higher than that of the Δcps strain for all of the alcohols tested.

### [Test example 9] (Evaluation of metal ion tolerance)

### • Evaluation of growth capacity in presence of metal ions

A copper oxide powder (CuO(II); nanopowder; < 50 nm particle size; Sigma) was added to an MRS liquid medium in an amount of 0.0025 to 0.01% (w/v). 0.05% (v/v) or 0.1% (v/v) of the MRS liquid culture for the stationary-phase YIT 9029 strain or Δcps strain, respectively, was inoculated into the medium containing CuO and cultured at 37°C (live bacterial counts at the start of culturing were approximately 1.5 × 10⁶ CFU/mL for both strains). The live bacterial count and the pH were measured over time until 24 hours elapsed. The fold change in growth was calculated, where a value of 1 was regarded as the live bacterial count at the start of culturing, and the average fold change in growth of five independent tests was calculated.

### • Measurement of live bacteria count

The live bacterial count was measured in the same manner as in test example 8.

### • Results

As shown in FIG. 14, in cases where no CuO was added, there was no difference in fold change in growth after 8 hours of culturing, and although the YIT 9029 strain exhibited a significant increase after 15 hours, the difference was approximately 2-fold. However, in cases where CuO was added, there was found to be a delay in growth of the Δcps strain, and there were significant differences in the 15-hour time point for every concentration tested. When CuO was added in concentrations of 0.0025% (w/v), 0.005% (w/v), and 0.01% (w/v), the fold change in growth of the YIT 9029 strain was approximately 4-fold, 9-fold, or 23-fold of that of the Δcps strain, respectively. At the 24-hour time point, when CuO was added in concentrations of 0.0025% (w/v) and 0.005% (w/v), the fold change in growth of the Δcps strain became comparable to that of the YIT 9029 strain up to a difference within 2-fold, but at a CuO concentration of 0.01% v, the fold change in growth of the Δcps strain was significantly different from that of the YIT 9029 strain from the 8-hour time point until the 24-hour time point, and the ratio of the fold changes in growth was such that the fold change in growth of the YIT 9029 strain was approximately 2-fold, 23-fold, or 6-fold of that of the Δcps strain at the respective time points.

From the matters above, it was apparent that the Δcps strain has a weaker tolerance to copper ions than the YIT 9029 strain.

In relation to pH, at the 15-hour time point of culturing for CuO concentrations of 0.005% (w/v) and 0.01% (w/v), where a large difference between the bacterial strains was present, the pH did not reach 4.8, which is the pH level that the growth of the Δcps strain would start to stagnate in the MRS medium. In particular, the average pH of the Δcps strain, where the maximum difference in the fold changes of growth was observed, was 5.8, which was at the 15-hour time point at a CuO concentration of 0.01% (w/v), which was a comparatively high pH value. Therefore, it was suggested that the difference in fold changes in growth between the bacterial strains was not caused by the difference in acid tolerance between the bacterial strains but due to the difference in tolerance to the presence of copper ions.

Separately, by microscopic observation, there was found to be a deformation in the form of the bacterial strains cultured in the presence of CuO (shrinkage or spherization) in substantially all cells of the Δcps strain at a CuO concentration of 0.0025% (w/v). However, in the YIT 9029 strain, no deformed cells were found at a CuO concentration of 0.0025% (w/v). Deformation in the YIT 9029 strain was first observed in a minor fraction (approximately 10%) of the cells at a CuO concentration of 0.005% (w/v), and marked deformation was found in substantially all cells at a CuO concentration of 0.01% (w/v).

### [Test example 10] (Evaluation of tolerance to surface-active agents)

### • Evaluation of growth capacity in presence of surface-active agents

Hexadecyltrimethylammonium bromide (CTAB; Sigma) and benzalkonium chloride (Takeda Pharmaceutical Co., Ltd.) serving as cationic surfactants were adjusted to a concentration of 0.63 to 80 µM through double stepwise dilution using pure water. Additionally, sodium lauryl sulfate (SDS) serving as an anionic surfactant was similarly adjusted to a concentration of 0.06 to 8 mM using pure water. The resulting solutions were dispensed into a 96-well plate in an amount of 50 µL/well. The MRS liquid medium in which the YIT 9029 strain or the Δcps strain was inoculated to approximately 1 × 10⁶ CFU/mL was added to the 96-well plate in an amount of 50 µL/well and cultured for 24 hours at 37°C. After culturing, the 96-well plate was visually observed from above and below, and the lowest concentration at which no precipitation due to bacterial cells occurred for each substance was regarded as the minimum inhibitory concentration (MIC). The geometric mean of the MIC values obtained through three independent tests was calculated.

### • Results

As shown in FIG. 15, in cases involving CTAB or benzalkonium chloride, which are cationic surfactants, the mean of the MIC for the YIT 9029 strain was 2.0 to 2.5 times that for the Δcps strain in both instances, and it was apparent that the Δcps strain has a weaker tolerance to cationic surfactants than the YIT 9029 strain. By contrast, in cases involving sodium lauryl sulfate (SDS), which is an anionic surfactant, the mean of the MIC for the YIT 9029 strain was 1.3-fold of that for the Δcps strain, and the tendency for the Δcps strain to have a weaker tolerance to anionic surfactants than the YIT 9029 strain was observed.

### [Test example 11] (Adhesion to hexadecane)

### • Treatment of bacterial cells with hexadecane

The stationary-phase YIT 9029 strain or Δcps strain that was cultured for 24 hours using the MRS liquid medium was centrifuged for 15 minutes at 10,000 × g to precipitate. The supernatant was removed, and a suspension was produced in an equivalent amount of a PUM buffer (16.9 g/L K₂HPO₄, 7.26 g/L KH₂PO₄, 1.8 g/L of urea, and 0.2 g/L of MgSO₄·7H₂O), after which centrifugation and suspension were repeated. The resulting suspension was diluted using the PUM buffer such that the turbidity (OD615) of the suspension was approximately 0.5 at a wavelength of 615 nm (maximum absorbance wavelength; measurement wavelength range: 575 to 660 nm), the turbidity being measured using a spectrophotometer PiCOSCOPE PAS-110 (Ushio Denki KK). 1.2 mL of the suspension was dispensed into a 15-mL sample tube, and 0.2 to 4.8 mL of hexadecane was added thereto from above. The resulting combination was stirred for 30 seconds using a vortex mixer and then allowed to stand for 30 minutes. After observation of the visual appearance of the resulting combination, the aqueous layer was collected, and the OD615 and total bacterial count were measured.

### • Measurement of total bacterial count

The liquid culture produced using the MRS liquid medium or the aqueous layer containing the bacteria was diluted 50-fold using phosphate-buffered saline, and 2.7 µL of the resulting diluted suspension was added to one frame of a glass slide (Matsunami Glass Ind., Ltd.) equipped with a water-repellent-paint frame measuring 1 cm × 1 cm. Cover glass was placed thereon, and the resulting product was imaged in 100 or more fields of view at 400× magnification using a digital microscope VHX-8000 (Keyence Corp.). The fields of view were joined, and bacterial cells within the area of one-sixth (16.65 mm²) of the entire frame (100 mm²) were automatically extracted and counted.

### • Results

In regard to the visual appearance after testing, emulsion droplets of the Δcps strain produced after the stirring were apparently larger than that of the YIT 9029 strain, the difference being visually perceptible. Additionally, it was found through visual assessment of the collected aqueous layer that there was a dramatic decrease in turbidity (increase in transparency) in the case of the Δcps strain compared to that of the YIT 9029 strain.

As shown in FIG. 16, there was found to be a decrease in the OD615 of the aqueous layer in association with an increase in the ratio of hexadecane to both bacterial strains, but the reduction was found to be more significant for the Δcps strain than for the YIT 9029 strain.

As shown in FIG. 17, upon investigation of the bacterial count in the aqueous layer, there was found to be a decrease in the bacterial count in the aqueous layer in association with an increase in the ratio of hexadecane to both bacterial strains, but the reduction was found to be more significant for the Δcps strain than for the YIT 9029 strain, in the same manner as with the OD615.

As a result of investigating where the bacterial cells in the Δcps strain migrated, the bacterial cells were found to accumulate at a high density in the interior of the emulsion droplets (i.e., in the aqueous layer), whereas the bacterial cells were substantially absent in the oil layer.

As described above, it was apparent that the affinity of the Δcps strain to hexadecane was higher than that of the YIT 9029 strain, which was presumably because the hydrophobicity of the bacterial cell surfaces was higher in the Δcps strain, without the cell-wall polysaccharides being formed.

### [Test example 12] (Measurement of zeta potential)

A pre-cultured suspension of the bacterial strains was inoculated in an amount of 1% (v/v) in the MRS liquid medium and cultured for 24 hours at 37°C. Next, a procedure comprising of centrifugation for 5 minutes at 10,000 × g and washing the resulting precipitate with ultra-pure water (Milli-Q) in an amount equivalent to that of the MRS liquid medium used in culturing was performed for 2 cycles. The turbidity (OD615) was adjusted using the ultra-pure water (Milli-Q) such that the turbidity as measured using the spectroscope PiCOSCOPE PAS-110 (Ushio Denki KK) was 1.0, and the resulting combination was regarded as a bacterial suspension. 0.2 mL of the bacterial suspension was added to 1.8 mL of a 5 mM potassium phosphate buffer solution (pH: 7.0, 4.8, or 4.0) immediately before the zeta potential was measured, and 1 mL of the resulting combination was filled into a capillary cell DTS1070 (Malvern Instruments Ltd.). The capillary cell was ultrasonicated for 15 seconds, after which three measurements were made for each sample using a Zetasizer Nano (Malvern Instruments Ltd.), and the average value of the zeta potential was calculated.

According to the results, there was no large difference between the two tests and, overall, the absolute value of the negative zeta potential dropped as the pH decreased, as shown in FIG. 18. The zeta potential fluctuated with the change in pH over a range of -0.8 to -4.7 mV for the YIT 9029 strain and over a range of -4.4 to -14.2 mV for the Δcps strain. The absolute value of the zeta potential for the Δcps strain at the same pH was consistently about 3 to 4 times greater than that for the YIT 9029 strain.

As described above, it was apparent that the fluctuation of the zeta potential toward negative values was greater in the Δcps strain than in the YIT 9029 strain, which was presumably because the properties of the bacterial cell surfaces in the Δcps strain, without the formation of the cell-wall polysaccharides, raised the amount of H+ ions attracted to the cells (reducing the pH around the bacterial cells).

### [Preparation example 1] (Construction of plasmid vector for introducing cps1 gene cluster)

There was constructed a plasmid vector into which the cell-wall-associated polysaccharide synthesis gene cluster (cps1 gene cluster) was incorporated.

Specifically, first, DNA corresponding to a genome region having a total length of approximately 11 kb was amplified through PCR in which the genome DNA of the *Lacticaseibacillus paracasei* YIT 9029 strain was used as a template, said DNA including the cps1 gene cluster composed of the ten genes described above. Table 4 shows a primer set that was used. The primer indicated by SEQ ID NO: 31 has a sequence of a region including a ribosome binding site sequence upstream of the cps1A gene, and a sequence including an SmaI cleavage site at the 5'-terminal side of the aforementioned sequence. The primer indicated by SEQ ID NO: 32 has a sequence that is complementary to the region downstream of the cps1J gene, and a sequence including a PstI cleavage site at the 5'-terminal side of the aforementioned sequence.

**[Table 4]**

| Table 4: Primer sequences | | | |
|---|---|---|---|
| Description of amplified/extended sequence | Primer orientation | Sequence (5' → 3') | SEQ ID NO |
| YIT 9029 strain genome: amplification of approximately 11 kb including cps1 gene cluster | Forward | 5'- TAACCCGGGTGGACTTGATTACACAAGC- 3' | SEQ ID NO: 31 |
| | Reverse | 5'- TAACTGCAGACACTCTTTTTACACTGCG- 3' | SEQ ID NO: 32 |

PCR was carried out using the aforementioned primer set in accordance with a product attachment method using KOD Plus DNA polymerase (made by Toyobo Co., Ltd.). After the reaction, phenol/chloroform extraction and ethanol precipitation processes were performed, and the amplified DNA was purified. The DNA was cleaved (20 hours at 37°C) using restriction enzymes SmaI and PstI (made by Takara Bio Inc.), after which the phenol/chloroform extraction and ethanol precipitation processes were performed again, and a DNA fragment composed of the cps1 gene cluster was purified.

pYAP300 was prepared as a plasmid vector. The plasmid vector was provided with phage-FSW-derived attP sites and int genes, configured to be site-specifically incorporated into the attB site on the genome of *Lacticaseibacillus casei.* The plasmid DNA was cleaved (20 hours at 37°C) using the restriction enzymes SmaI and PstI (made by Takara Bio Inc.) and configured in linear form. After the reaction, the phenol/chloroform extraction and ethanol precipitation processes were performed, and the linearized plasmid DNA was purified. Water was subsequently added to 20 µL of a 10X calf intestinal phosphatase (CIP) buffer (made by Toyobo Co., Ltd.) to bring the volume to 200 µL, 3 µL of CIP (made by Toyobo Co., Ltd.) was added, the resulting combination was incubated for 2 hours at 37°C, and the terminals were dephosphorylated. After the reaction, the phenol/chloroform extraction and ethanol precipitation processes were performed, and the linearized plasmid DNA was purified.

The aforementioned DNA fragment composed of the cps1 gene cluster, and pYAP300, were ligated in the manner described below. The DNA fragment composed of the cps1 gene cluster and the linearized pYAP300 plasmid DNA were mixed together in amounts of about 0.01 to 0.1 µg each, an equivalent volume of Solution I of DNA Ligation Kit ver. 2.1 (made by Takara Bio Inc.) was added to the resulting mixture, and the resulting combination was incubated for 30 minutes at 16°C and then placed in ice. The DNA was bound in the manner described above such that the genes from the cps1A gene to the cps1J gene were inserted downstream of a transcription promoter in a pYAP300 vector, the bound DNA was added to competent cells of *Escherichia coli* JM109, transformation was carried out, and the resulting product was applied to an LB agar culture medium containing 500 µg/mL of erythromycin and allowed to stand for 2 days at 37°C. Plasmids were extracted from the resulting colony, and a target recombinant plasmid was confirmed to have been obtained according to the size of the extracted plasmids and the size of the fragment produced through cleavage by the restriction enzymes.

### [Preparation example 2] (Development of strain into which cps1 gene cluster is introduced)

The cps1 gene cluster was introduced into *Lacticaseibacillus casei* ATCC334 using the cps1 gene cluster introduction vector obtained in preparation example 1.

Specifically, *Lacticaseibacillus casei* ATCC334 was propagated in the MRS liquid medium, the resulting culture solution was centrifuged for 5 minutes at 5,000 × g and 4°C in a logarithmic growth phase and collected, the bacterial cells were washed once each with ice - cooled 20 mM HEPES (pH: 7.0) and 10% glycerol, and the washed bacterial cells were suspended in (Klett value of initial culture solution) × 2 µL of 10% glycerol. 50 µL of the bacterial suspension and 3 µL of the recombinant plasmid DNA solution were mixed, the resulting mixture was charged into a 2-mm- wide electroporation cuvette, and electroporation was carried out at 1.5 kV, 200 Ω, and a capacitance of 25 µF using the Gene Pulser II (Bio Rad Laboratories, Inc.).

After the electroporation, 1 mL of the MRS liquid medium was added, culturing was performed for 2 hours at 37°C, the cultured product was applied to an MRS agar medium to which erythromycin at a concentration of 20 µg/mL was added and incubated for 2 to 3 days at 37°C, and a colony having erythromycin resistance was obtained. Some of the resulting colonies were sampled, and confirmation of whether the target plasmid was inserted at the target position was carried out through colony PCR using a primer having a sequence selected from regions near the attB site on the genome of *Lacticaseibacillus casei* ATCC334 and a primer having a sequence selected from regions that are comparatively near the terminal of the DNA prepared in preparation example 1.

Bacterial cells derived from the *Lacticaseibacillus casei* ATCC334 obtained in the manner described above were cps1-gene-cluster-introduced strains including, at the attB site on the genome thereof, the ten genes described above, i.e., the cps1A gene (SEQ ID NO: 1), the cps1B gene (SEQ ID NO: 2), the cps1C gene (SEQ ID NO: 3), the cps1D gene (SEQ ID NO: 4), the cps1E gene (SEQ ID NO: 5), the cps1F gene (SEQ ID NO: 6), the cpslG gene (SEQ ID NO: 7), the cps1H gene (SEQ ID NO: 8), the cpslI gene (SEQ ID NO: 9), and the cps1J gene (SEQ ID NO: 10), said genes being acquired from the YIT 9029 strain.

## Claims

1. A composition for imparting environmental stress tolerance to bacteria, the composition including a cps1 gene cluster derived from *Lacticaseibacillus paracasei.*

2. The composition according to claim 1, wherein the cps1 gene cluster includes a cps1A gene, a cps1B gene, a cps1C gene, a cps1D gene, a cps1E gene, a cps1F gene, a cpslG gene, a cps1H gene, a cps1I gene, and a cps1J gene.

3. The composition according to claim 2, wherein
the cps1A gene has the polynucleotide sequence indicated by SEQ ID NO: 1 or a polynucleotide sequence that is at least 90% homologous thereto,
the cps1B gene has the polynucleotide sequence indicated by SEQ ID NO: 2 or a polynucleotide sequence that is at least 90% homologous thereto,
the cps1C gene has the polynucleotide sequence indicated by SEQ ID NO: 3 or a polynucleotide sequence that is at least 90% homologous thereto,
the cps1D gene has the polynucleotide sequence indicated by SEQ ID NO: 4 or a polynucleotide sequence that is at least 90% homologous thereto,
the cps1E gene has the polynucleotide sequence indicated by SEQ ID NO: 5 or a polynucleotide sequence that is at least 90% homologous thereto,
the cps1F gene has the polynucleotide sequence indicated by SEQ ID NO: 6 or a polynucleotide sequence that is at least 90% homologous thereto,
the cps1G gene has the polynucleotide sequence indicated by SEQ ID NO: 7 or a polynucleotide sequence that is at least 90% homologous thereto,
the cps1H gene has the polynucleotide sequence indicated by SEQ ID NO: 8 or a polynucleotide sequence that is at least 90% homologous thereto,
the cps1I gene has the polynucleotide sequence indicated by SEQ ID NO: 9 or a polynucleotide sequence that is at least 90% homologous thereto, and
the cps1J gene has the polynucleotide sequence indicated by SEQ ID NO: 10 or a polynucleotide sequence that is at least 90% homologous thereto.

4. The composition according to claim 2, wherein
the cps1A gene codes the amino acid sequence indicated by SEQ ID NO: 11 or an amino acid sequence that is at least 90% homologous thereto,
the cps1B gene codes the amino acid sequence indicated by SEQ ID NO: 12 or an amino acid sequence that is at least 90% homologous thereto,
the cps1C gene codes the amino acid sequence indicated by SEQ ID NO: 13 or an amino acid sequence that is at least 90% homologous thereto,
the cps1D gene codes the amino acid sequence indicated by SEQ ID NO: 14 or an amino acid sequence that is at least 90% homologous thereto,
the cps1E gene codes the amino acid sequence indicated by SEQ ID NO: 15 or an amino acid sequence that is at least 90% homologous thereto,
the cps1F gene codes the amino acid sequence indicated by SEQ ID NO: 16 or an amino acid sequence that is at least 90% homologous thereto,
the cps1G gene codes the amino acid sequence indicated by SEQ ID NO: 17 or an amino acid sequence that is at least 90% homologous thereto,
the cps1H gene codes the amino acid sequence indicated by SEQ ID NO: 18 or an amino acid sequence that is at least 90% homologous thereto,
the cpslI gene codes the amino acid sequence indicated by SEQ ID NO: 19 or an amino acid sequence that is at least 90% homologous thereto, and
the cps1J gene codes the amino acid sequence indicated by SEQ ID NO: 20 or an amino acid sequence that is at least 90% homologous thereto.

5. The composition according to any one of claims 1 to 4, wherein the cps1 gene cluster is used in a form of being incorporated into a vector.

6. The composition according to any one of claims 1 to 4, wherein the environmental stress against which tolerance is to be imparted is caused by at least one selected from the group consisting of acids, bile components, alcohols, metal ions, hydrophobic substances, and surface-active agents.

7. A method for imparting environmental stress tolerance to bacteria, **characterized by** including a step in which the cps1 gene cluster is introduced into designated bacteria using the composition according to any one of claims 1 to 4.

8. The method for imparting environmental stress tolerance to bacteria according to claim 7, wherein the method includes imparting tolerance to environmental stress caused by at least one selected from the group consisting of acids, bile components, alcohols, metal ions, hydrophobic substances, and surface-active agents.
